# EUROPEAN PATENT APPLICATION

(11) **EP 1 354 880 A1**
(43) Date of publication of application: **22.10.2003**
(21) Application number: 02710353.0
(22) Date of filing: 25.01.2002
(51) Int. Cl.: C07D 277/46, C07D 417/14, C07D 277/60, C07D 277/84, C07D 513/04, A61K 31/427, A61K 31/4439, A61K 31/5377, A61K 31/428, A61K 31/429, A61P 43/00, A61P 7/04, A61P 7/02

(54) **HALOGEN COMPOUNDS HAVING THROMBOPOIETIN RECEPTOR AGONISM**

(30) Priority: 26.01.2001 JP 2001017785; 24.07.2001 JP 2001223413
(71) Applicant: SHIONOGI & CO., LTD., Osaka-shi, Osaka 541-0045 (JP)
(72) Inventor: TAKEMOTO, Hiroshi, Shionogi & Co., Ltd., Osaka-shi, Osaka 553-0002 (JP); TAKAYAMA, Masami, Shionogi & Co., Ltd., Osaka-shi, Osaka 553-0002 (JP); SHIOTA, Takeshi, Shionogi & Co., Ltd., Osaka-shi, Osaka 553-0002 (JP)
(74) Representative: VOSSIUS & PARTNER
(86) International application number: JP0200547
(87) International publication number: WO02059100

(57) **Abstract**

Compounds of the general formula (III), prodrugs thereof, pharmaceutically acceptable salts of both, or solvates of them and exhibiting thrombopoietin receptor agonism: 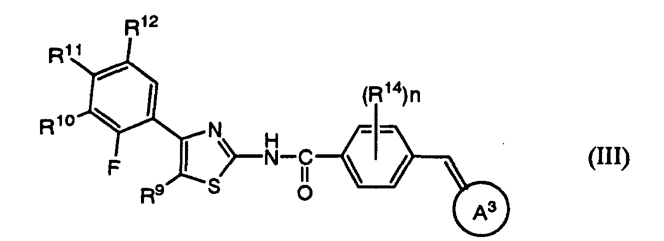 wherein R⁹ is hydrogen atom, optionally substituted lower alkyl, or the like; R¹⁰, R¹¹ and R¹² are each independently optionally substituted alkyl, halogen atom, or the like; R¹⁴ is each independently lower alkyl, halogen atom, or the like; n is an integer of 0 to 2; A³ is a group represented by the formula: 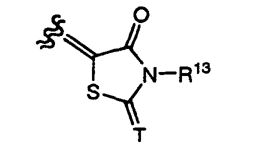 wherein R¹³ is hydrogen atom or lower alkyl; T is oxygen atom or sulfur atom.

## Description

### Technical Field

The present invention relates to a pharmaceutical composition exhibiting thrombopoietin receptor agonism.

### Background Art

Thrombopoietin, polypeptide cytokine composed of 332 amino acids, activates the production of platelets by stimulating the differentiation and proliferation of megakaryocytes through the receptor and is expected as a medicine for hemopathy accompanied with the unusual number of platelets, for example, thrombocytopenia. DNA sequences encoding the thrombopoietin receptor have been described in Proc. Natl. Acad. Sci., 89, 5640-5644 (1992). Low molecular peptides having an affinity for the thrombopoietin receptor is also known (JP98/72492A and WO96/40750), but these peptide derivatives are not generally practical for oral administration.

1,4-Benzodiazepine derivatives as a low molecule compound having an affinity to the thrombopoietin receptor is described in JP99/1477A and JP99/152276A.

The compound having a similar structure of the present invention compound are described in EP 389699 A1, WO97/32863, J. Med. Chem. 25, 1992, 1853-1864, JP92/99770, and the like, but the affinity for thrombopoietin receptor is not described therein.

### Disclosure of Invention

The object of the present invention is to prepare pharmaceutical compositions exhibiting thrombopoietin receptor agonism and provide orally administrable platelet production modifiers.

In the above situation, the inventors of the present invention have found that the following compounds exhibit strong thrombopoietin receptor agonism to achieve the present invention.

The present invention relates to:
1) A pharmaceutical composition exhibiting thrombopoietin receptor agonism which contains as an active ingredient a compound of the general formula (I): 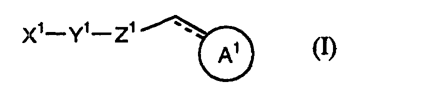 wherein X¹ is a group represented by the formula: 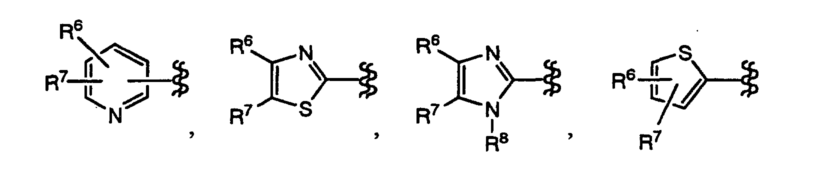 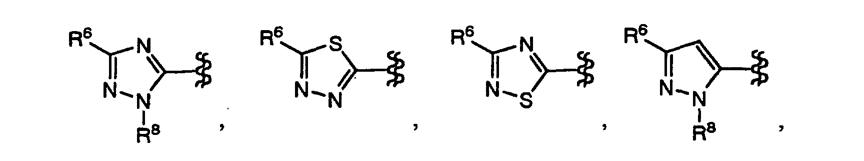 or 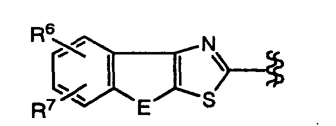 wherein E is -CH₂-, -(CH₂)₂-, -(CH₂)₃-, -O-CH₂-, or -S-CH₂-; one of R⁶ and R⁷ is a group represented by the formula: 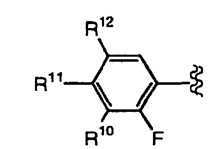 wherein R¹⁰, R¹¹, and R¹² are each independently hydrogen atom, alkyl optionally substituted with one or more substituent(s) selected from substituent group A, cycloalkyl, alkyloxy optionally substituted with one or more substituent(s) selected from substituent group A, alkylthio, halogen atom, phenyl optionally substituted with one or more substituent(s) selected from substituent group B, heteroaryl optionally substituted with one or more substituent(s) selected from substituent group B, or non-aromatic heterocyclic group optionally substituted with one or more substituent(s) selected from substituent group B,
   substituent group A consists of cycloalkyl, hydroxy, optionally substituted alkyloxy, halogen atom, carboxy, lower alkyloxycarbonyl, aryloxycarbonyl, optionally substituted amino, optionally substituted aminocarbonyl, phenyl optionally substituted with one or more substituent(s) selected from substituent group B, non-aromatic heterocyclic group, and heteroaryl,
   substituent group B consists of hydroxy, alkyl, halogen atom, halo(lower)alkyl, carboxy, lower alkyloxycarbonyl, alkyloxy, optionally substituted amino, non-aromatic heterocyclic group, and heteroaryl;
   the other of R⁶ and R⁷ is hydrogen atom, optionally substituted lower alkyl, carboxy, lower alkyloxycarbonyl, halogen atom, optionally substituted aminocarbonyl, optionally substituted heteroaryl, or optionally substituted aryl; R⁸ is hydrogen atom or lower alkyl;
   Y¹ is -NR^{A}CO-(CR^{C}R^{D})₀₋₂-, -NR^{A}CO-(CH₂)₀₋₂-V-, -NR^{A}CO-CR^{C}=CR^{D}-, -V-(CH₂)₁₋ ₅-NR^{A}CO-(CH₂)₀₋₂-, -V-(CH₂)₁₋₅-CONR^{A}-(CH₂)₀₋₂, -CONR^{A}-(CH₂)₀₋₂-,-(CH₂)₀₋₂-NR^{A}-SO₂-(CH₂)₀₋₂-, -(CH₂)₀₋₂-SO₂-NR^{A}-(CH₂)₀₋₂-, -NR^{A}-(CH₂)₀₋₂-, -NR^{A}-CO-NR^{A}-, -NR^{A}-CS-NR^{A}-,-N=C(-SR^{A})-NR^{A}-, -NR^{A}CSNR^{A}CO-, -N=C(-SR^{A})-NR^{A}CO-, -NR^{A}-(CH₂)₁₋₂-NR^{A}-CO-,-NR^{A}CONR^{A}NR^{B}CO-, or -N=C(-NR^{A}R^{A})-NR^{A}-CO- wherein R^{A} is each independently hydrogen atom or lower alkyl; R^{B} is hydrogen atom or phenyl; R^{C} and R^{D} are each independently hydrogen atom, halogen atom, optionally substituted lower alkyl, optionally substituted lower alkyloxy, optionally substituted lower alkylthio, optionally substituted lower alkenyl, optionally substituted lower alkynyl, optionally substituted aryl, optionally substituted heteroaryl, optionally substituted cycloalkyl, optionally substituted aralkyl, optionally substituted heteroarylalkyl, optionally substituted non-aromatic heterocyclic group, or optionally substituted amino; V is oxygen atom or sulfur atom;
   Z¹ is optionally substituted arylene, optionally substituted heteroarylene, optionally substituted non-aromatic heterocyclicdiyl, or optionally substituted cycloalkylene;
   A¹ ring is a ring represented by the formula: 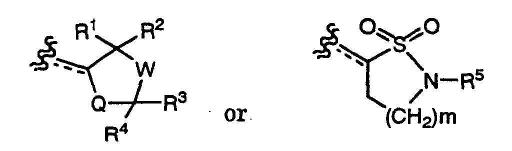 wherein R¹ and R² are hydrogen atom or taken together to be oxygen atom or sulfur atom; R³ and R⁴ are hydrogen atom or taken together to be oxygen atom or sulfur atom; R⁵ is hydrogen atom or lower alkyl; Q and W are each independently -O-, -S-, -N(R^{F})-wherein R^{F} is hydrogen atom or lower alkyl, or -CH₂-; m is 1,2, or 3; a broken line (---) represents the presence or absence of a bond;
   a broken line (---) represents the presence or absence of a bond;
   provided that R¹⁰, R¹¹, and R¹² are not hydrogen atom at the same time; when R¹⁰ and R¹¹ are hydrogen, R¹² is not fluoro; when R¹⁰ is hydrogen, R¹¹ and R¹² are not fluoro;
   its prodrug, or their pharmaceutically acceptable salt, or solvate thereof.
   More details, the present invention relates to the followings 2) to 21).
2) A pharmaceutical composition exhibiting thrombopoietin receptor agonism of 1), wherein Y¹ is -NHCO-, -CONH-, -NHCH₂-, -NHCO-CH=CH-, or -NHSO₂-.
3) A pharmaceutical composition exhibiting thrombopoietin receptor agonism of 1) or 2), wherein Z¹ is halogen atom or 1,4-phenylene optionally substituted with lower alkyl.
4) A pharmaceutical composition exhibiting thrombopoietin receptor agonism which contains a compound of any one of 1) to 3), wherein A¹ ring is a group represented by the formula: 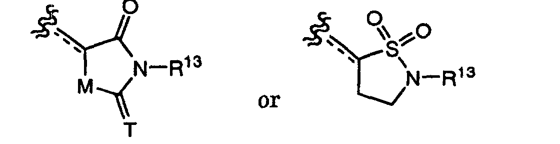 wherein R¹³ is hydrogen atom or lower alkyl; M is -S-, -O-, -N(R^{E})- wherein R^{E} is hydrogen atom or lower alkyl; or -CH₂-; T is oxygen atom or sulfur atom; a broken line (---) represents the presence or absence of a bond.
5) A pharmaceutical composition exhibiting thrombopoietin receptor agonism of any one of 1) to 4), wherein a broken line (---) represents the presence of a bond.
6) A pharmaceutical composition exhibiting thrombopoietin receptor agonism of any one of 1) to 5), which is a platelet production modifier.
7) Use of a compound of any one of 1) to 5), for preparation of a medicine for modifying platelet production.
8) A method for modifying platelet production of a mammal, including a human, which comprises administration to said mammal of a compound of any one of 1) to 5) in a therapeutically effective amount.
9) A compound represented by the general formula (II): 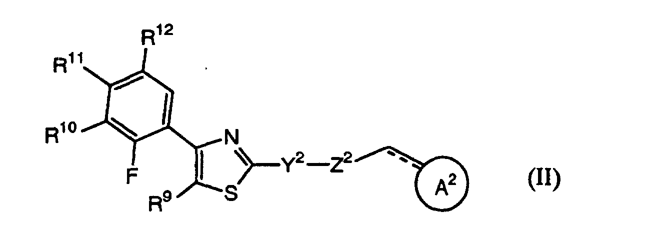 wherein R⁹ is hydrogen atom, optionally substituted lower alkyl; carboxy, lower alkyloxycarbonyl, halogen atom, or optionally substituted aminocarbonyl;
   R¹⁰, R¹¹, and R¹² are each independently hydrogen atom, alkyl optionally substituted with one or more substituent(s) selected from substituent group A, cycloalkyl, alkyloxy optionally substituted with one or more substituent(s) selected from substituent group A, alkylthio, halogen atom, phenyl optionally substituted with one or more substituent(s) selected from substituent group B, heteroaryl optionally substituted with one or more substituent(s) selected from substituent group B, or non-aromatic heterocyclic group optionally substituted with one or more substituent(s) selected from substituent group B,
   substituent group A consists of cycloalkyl, hydroxy, optionally substituted alkyloxy, halogen atom, carboxy, lower alkyloxycarbonyl, aryloxycarbonyl, optionally substituted amino, optionally substituted aminocarbonyl, phenyl optionally substituted with one or more substituent(s) selected from substituent group B, non-aromatic heterocyclic group, and heteroaryl,
   substituent group B consists of hydroxy, alkyl, halogen atom, halo(lower)alkyl, carboxy, lower alkyloxycarbonyl, alkyloxy, optionally substituted amino, non-aromatic heterocyclic group, and heteroaryl;
   Y² is -NR^{A}CO-(CR^{C}R^{D})₀₋₂-, -NR^{A}CO-(CH₂)₀₋₂-V-, -NR^{A}CO-CR^{C}=CR^{D}-, -V-(CH₂)₁₋₅-NR^{A}CO-(CH₂)_{0.2}-, -V-(CH₂)₁₋₅-CONR^{A}-(CH₂)₀₋₂-, -CONR^{A}-(CH₂)₀₋₂-, -(CH₂)₀₋₂-NR^{A}-SO₂-(CH₂)₀₋₂-, -(CH₂)₀₋₂-SO₂-NR^{A}-(CH₂)₀₋₂-, -NR^{A}-(CH₂)₀₋₂-, -NR^{A}-CO-NR^{A}-, -NR^{A}-CS-NR^{A}-,-N=C(-SR^{A})-NR^{A}-, -NR^{A}CSNR^{A}CO-, -N=C(-SR^{A})-NR^{A}CO-, -NR^{A}-(CH₂)₁₋₂-NR^{A}-CO-,-NR^{A}CONR^{A}NR^{B}CO-, or -N=C(-NR^{A}R^{A})-NR^{A}-CO- wherein R^{A} is each independently hydrogen atom or lower alkyl; R^{B} is hydrogen atom or phenyl; R^{C} and R^{D} are each independently hydrogen atom, halogen atom, optionally substituted lower alkyl, optionally substituted lower alkyloxy, optionally substituted lower alkylthio, optionally substituted lower alkenyl, optionally substituted lower alkynyl, optionally substituted aryl, optionally substituted heteroaryl, optionally substituted cycloalkyl, optionally substituted aralkyl, optionally substituted heteroarylalkyl, optionally substituted non-aromatic heterocyclic group, or optionally substituted amino; V is oxygen atom or sulfur atom;
   Z² is optionally substituted phenylene, optionally substituted 2,5-pyridinediyl, optionally substituted 2,5-thiophenediyl, or optionally substituted 2,5-furandiyl;
   A² ring is a ring represented by the formula: 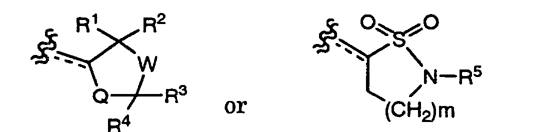 wherein R¹ and R² are hydrogen atom or taken together to be oxygen atom or sulfur atom; R³ and R⁴ are hydrogen atom or taken together to be oxygen atom or sulfur atom; R⁵ is hydrogen atom or lower alkyl; Q and W are each independently -O-, -S-, -N(R^{F})-wherein R^{F} is hydrogen atom or lower alkyl, or -CH₂-; m is 1,2, or 3; a broken line (---) represents the presence or absence of a bond;
   a broken line (---) represents the presence or absence of a bond;
   provided that R¹⁰, R¹¹, and R¹² are not hydrogen atom at the same time; when R¹⁰ and R" are hydrogen, R¹² is not fluoro; when R¹⁰ is hydrogen, R¹¹ and R¹² are not fluoro;
   its prodrug, or their pharmaceutically acceptable salt, or solvate thereof.
10) A compound of 9), wherein Y² is -NHCO-, -CONH-, -NHCH₂-, -NHCO-CH=CH-, or -NHSO₂-;
   its prodrug, or their pharmaceutically acceptable salt, or solvate thereof.
11) A compound of 9), wherein Y² is -NHCO-;
   its prodrug, or their pharmaceutically acceptable salt, or solvate thereof.
12) A compound of any one of 9) to 11), wherein Z² is halogen atom or 1,4-phenylene optionally substituted with lower alkyl;
   its prodrug, or their pharmaceutically acceptable salt, or solvate thereof.
13) A compound of any one of 9) to 12), wherein A² ring is a group represented by the formula: 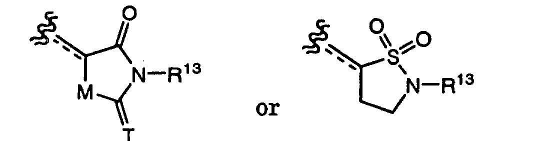 wherein R¹³ is hydrogen atom or lower alkyl; M is -S-, -O-, -N(R^{E})- wherein R^{E} is hydrogen atom or lower alkyl; or -CH₂-; T is oxygen atom or sulfur atom; a broken line (---) represents the presence or absence of a bond; its prodrug, or their pharmaceutically acceptable salt, or solvate thereof.
14) A compound of any one of 9) to 13), wherein a broken line (---) represents the presence of a bond;
   its prodrug, or their pharmaceutically acceptable salt, or solvate thereof.
15) A compound of the general formula (III): 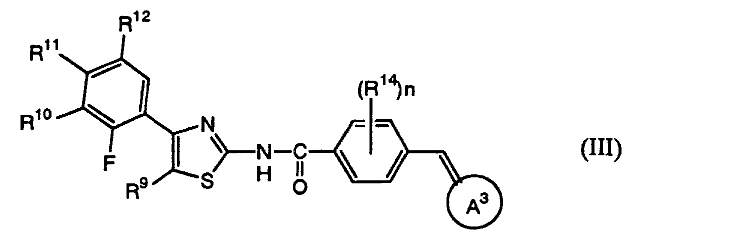 wherein R⁹ is hydrogen atom, optionally substituted lower alkyl; carboxy, lower alkyloxycarbonyl, halogen atom, or optionally substituted aminocarbonyl;
   R¹⁰ is alkyl optionally substituted with one or more substituent(s) selected from substituent group A, cycloalkyl, alkyloxy optionally substituted with one or more substituent(s) selected from substituent group A, alkylthio, halogen atom, phenyl optionally substituted with one or more substituent(s) selected from substituent group B, heteroaryl optionally substituted with one or more substituent(s) selected from substituent group B, or non-aromatic heterocyclic group optionally substituted with one or more substituent(s) selected from substituent group B,
   R¹¹ and R¹² are each independently hydrogen atom, alkyl optionally substituted with one or more substituent(s) selected from substituent group A, cycloalkyl, alkyloxy optionally substituted with one or more substituent(s) selected from substituent group A, alkylthio, halogen atom, phenyl optionally substituted with one or more substituent(s) selected from substituent group B, heteroaryl optionally substituted with one or more substituent(s) selected from substituent group B, or non-aromatic heterocyclic group optionally substituted with one or more substituent(s) selected from substituent group B,
   substituent group A consists of cycloalkyl, hydroxy, optionally substituted alkyloxy, halogen atom, carboxy, lower alkyloxycarbonyl, aryloxycarbonyl, optionally substituted amino, optionally substituted aminocarbonyl, phenyl optionally substituted with one or more substituent(s) selected from substituent group B, non-aromatic heterocyclic group, and heteroaryl,
   substituent group B consists of hydroxy, alkyl, halogen atom, halo(lower)alkyl, carboxy, lower alkyloxycarbonyl, alkyloxy, optionally substituted amino, non-aromatic heterocyclic group, and heteroaryl;
   R¹⁴ is each independently lower alkyl, halogen atom, halo(lower)alkyl, lower alkyloxy, halo(lower)alkyloxy, or hydroxy;
   n is an integer of 0 to 2;
   A³ ring is a group represented by the formula: 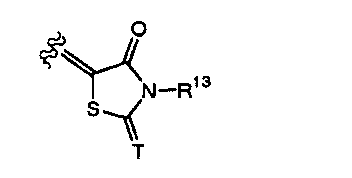 wherein R¹³ is hydrogen atom or lower alkyl; T is oxygen atom or sulfur atom; its prodrug, or their pharmaceutically acceptable salt, or solvate thereof.
16) A compound of 15), wherein R¹⁰ is alkyl optionally substituted with one or more substituent(s) selected from substituent group A, alkyloxy, halo(lower)alkyloxy, or phenyl optionally substituted with one or more substituent(s) selected from substituent group B; R¹¹ is hydrogen atom, halo(lower)alkyl, or halo(lower)alkyloxy; R¹² is hydrogen atom or fluoro;
   substituent group A consists of cycloalkyl, hydroxy, optionally substituted alkyloxy, halogen atom, carboxy, lower alkyloxycarbonyl, aryloxycarbonyl, optionally substituted amino, optionally substituted aminocarbonyl, phenyl optionally substituted with one or more substituent(s) selected from substituent group B, non-aromatic heterocyclic group, and heteroaryl,
   substituent group B consists of hydroxy, alkyl, halogen atom, halo(lower)alkyl, carboxy, lower alkyloxycarbonyl, alkyloxy, optionally substituted amino, non-aromatic heterocyclic group, and heteroaryl; its prodrug, or their pharmaceutically acceptable salt, or solvate thereof.
17) A pharmaceutical composition containing as an active ingredient a compound of any one of 9) to 16).
18) A pharmaceutical composition exhibiting thrombopoietin receptor agonism, which contains as an active ingredient a compound of any one of 9) to 16).
19) A platelet production modifier containing as an active ingredient a compound of any one of 9) to 16).
20) Use of a compound of any one of 9) to 16), for preparation of a medicine for modifying platelet production.
21) A method for modifying platelet production of a mammal, including a human, which comprises administration to said mammal of a compound of any one of 9) to 16) in a pharmaceutically effective amount..

In the present specification, the term "halogen" means fluoro, chloro, bromo, and iodo. Preferable are fluoro, chloro and bromo.

In the present specification, the term "alkyl" employed alone or in combination with other terms means a straight or branched chain monovalent hydrocarbon group having 1 to 15 carbon atom(s). Examples of alkyl include methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, n-nonanyl, n-decanyl, n-undecanyl, n-dodecanyl, n-tridecanyl, n-tetradecanyl, n-pentadecanyl, and the like. C1 to C10 alkyl is preferred. C1 to C6 alkyl is more preferred.

In the present specification, the term "lower alkyl" employed alone or in combination with other terms means a straight or branched chain monovalent hydrocarbon group having 1 to 8 carbon atom(s). Examples of lower alkyl include methyl, ethyl, n-propyl, isopropyl, n-butyl, isobutyl, sec-butyl, tert-butyl, n-pentyl, isopentyl, neo-pentyl, n-hexyl, isohexyl, n-heptyl, n-octyl, and the like. C1 to C6 alkyl is preferred. C1 to C3 alkyl is more preferred.

In the present specification, the term "cycloalkyl" employed alone or in combination with other terms means a cycloalkyl having 3 to 8 carbon atoms. Examples of cycloalkyl include cyclopropyl, cyclobutyl, cyclopentyl, cyclohexyl, cycloheptyl, cyclooctyl. C3 to C6 cycloalkyl is preferred.

The term "lower alkenyl" in the present specification means a straight or branched chain monovalent hydrocarbon group having 2 to 8 carbon atoms and one or more double bond. Examples of lower alkenyl include vinyl, allyl, 1-propenyl, 2-propenyl, a variety of butenyl isomers and the like. C2 to C6 alkenyl is preferred. C2 to C4 alkenyl is more preferred.

The term "lower alkynyl" used in the present specification means a straight or branched chain monovalent hydrocarbon group having 2 to 8 carbon atoms and one or more triple bond. Examples of lower alkynyl include ethynyl, 1-propynyl, 2-propynyl, 1-butynyl, 2-butynyl, 3-butynyl, 1-pentynyl, 2-pentynyl, a variety of pentynyl isomers and the like. C2 to C6 alkynyl is preferred. C2 to C4 alkynyl is more preferred.

In the present specification, the term "aryl" employed alone or in combination with other terms means monocyclic or condensed cyclic aromatic hydrocarbon. Examples of aryl include phenyl, 1-naphtyl, 2-naphtyl, anthryl, and the like.

The term "aralkyl" herein used means the above mentioned "lower alkyl" substituted with one or more of the above mentioned "aryl" at any possible position. Examples of the aralkyl are benzyl, phenethyl (e.g., 2-phenethyl and the like), phenylpropyl (e.g., 3-phenylpropyl and the like), naphthylmethyl (e.g., 1-naphthylmethyl, 2-naphthylmethyl, and the like), anthrylmethyl (e.g., 9-anthrylmethyl), and the like. Benzyl and phenylethyl are preferred.

In the present specification, the term "non-aromatic heterocyclic group" employed alone or in combination with other terms means a 5- to 7-membered non-aromatic ring which contains one or more heteroatoms selected from the group consisting of oxygen, sulfur, and nitrogen atoms in the ring and the 5- to 7-membered non-aromatic ring group may be condensed with two or more rings. Examples of the non-aromatic heterocyclic group are pyrrolidinyl (e.g., 1-pyrrolidinyl, 2-pyrrolidinyl), pyrrolinyl (e.g., 3-pyrrolinyl), imidazolidinyl (e.g., 2-imidazolidinyl), imidazolinyl (e.g., imidazolinyl), pyrazolidinyl (e.g., 1-pyrazolidinyl, 2-pyrazolidinyl), pyrazolinyl (e.g., pyrazolinyl), piperidinyl (e.g., piperidino, 2-piperidinyl), piperazinyl (e.g., 1-piperazinyl), indolynyl (e.g., 1-indolynyl), isoindolinyl (e.g., isoindolinyl), morpholinyl (e.g., morpholino, 3-morpholinyl), tetrahydrofuranyl, tetrahydropyranyl, and the like.

Preferable are morpholino, piperazino, pyrrolidino, teterahydrofuranyl, tetrahydropyranyl, and the like as "non-aromatic heterocyclic group" for R¹⁰, R¹¹, and R¹².

Preferable are morpholino, piperazino, piperidino, teterahydrofuranyl, tetrahydropyranyl, and the like as "non-aromatic heterocyclic group" for substituent group A.

Preferable are morpholino, piperazino, piperidino, pyrrolidino, teterahydrofuranyl, tetrahydropyranyl, and the like as "non-aromatic heterocyclic group" for substituent group B.

In the present specification, the term "heteroaryl" employed alone or in combination with other terms means a 5- to 6-membered aromatic heterocyclic group which contains one or more heteroatoms selected from the group consisting of oxygen, sulfur, and nitrogen atoms in the ring and may be fused with above mentioned "cycloalkyl", above mentioned "aryl", above mentioned "non-aromatic heterocyclic group", and other heteroaryl at any possible position. The heteroaryl, monocyclic or fused ring, may be bonded at any possible position. Examples of the heteroaryl are pyrrolyl (e.g., 1-pyrrolyl, 2-pyrrolyl, 3-pyrrolyl), furyl (e.g., 2-furyl, 3-furyl), thienyl (e.g., 2-thienyl 3-thienyl), imidazolyl (e.g., 2- imidazolyl, 4- imidazolyl), pyrazolyl (e.g., 1-pyrazolyl, 3-pyrazolyl), isothiazolyl (e.g., 3-isothiazolyl), isoxazolyl (e.g., 3-isoxazolyl), oxazolyl (e.g., 2-oxazolyl), thiazolyl (e.g., 2-thiazolyl), pyridyl (e.g., 2-pyridyl, 3-pyridyl, 4-pyridyl), pyrazinyl (e.g., 2-pyrazinyl), pyrimidinyl (e.g., 2-pyrimidinyl, 4-pyrimidinyl), pyridazinyl (e.g., 3-pyridazinyl), tetrazolyl(e.g., 1H-tetrazolyl), oxadiazolyl (e.g., 1,3,4-oxadiazolyl), thiadiazolyl (e.g., 1,3,4- thiadiazolyl), indolizinyl (e.g., 2-indolizinyl, 6-indolizinyl), isoindolyl (2-isoindolyl), indolyl (e.g., 1-indolyl, 2-indolyl, 3-indolyl), indazolyl (e.g., 3-indazolyl), puriyl (e.g., 8-puriyl), quinolizinyl ( e.g., 2-quinolizinyl), isoquinolyl (e.g., 3-isoquinolyl), quinolyl (e.g., 3-quinolyl, 5-quinolyl), phthalazinyl (e.g., 1-phthalazinyl), naphthyridinyl (e.g., 2-naphthyridinyl), quinolanyl (2-quinolanyl), quinazolinyl (e.g., 2-quinazolinyl), cinnolinyl (e.g., 3-cinnolinyl), pteridinyl (e.g., 2-pteridinyl), carbazolyl (e.g., 2-carbazolyl, 4-carbazolyl), phenanthridinyl (e.g., 2-phenanthridinyl, 3-phenanthridinyl), acridinyl (e.g., 1-acridinyl, 2-acridinyl), dibenzofuranyl (e.g., 1-dibenzofuranyl, 2-dibenzofuranyl), benzimidazolyl (e.g., 2-benzimidazolyl), benzisoxazolyl (e.g., 3-benzisoxazolyl), benzoxazolyl (e.g., 2-benzoxazolyl), benzoxadiazolyl (e.g., 4- benzoxadiazolyl), benzisothiazolyl (e.g., 3-benzisothiazolyl), benzothiazolyl (e.g., 2-benzothiazolyl), benzofuryl (e.g., 3-benzofuryl), benzothienyl (e.g., 2-benzothienyl), and the like.

Preferable are pyridyl, thienyl, furyl, pyrimidinyl, imidazolyl, thiazolyl, oxazolyl, triazolyl, and the like as "heteroaryl" for R¹⁰, R¹¹ and R¹².

Preferable are pyridyl, pyrazolyl, pyrimidinyl, imidazolyl, oxazolyl, thiazolyl, furyl, thienyl, and the like as "heteroaryl" for substituent A group.

Preferable are pyridyl, pyrazolyl, imidazolyl, and the like as "heteroaryl" for substituent B group.

The term "heteroarylalkyl" herein used means the above mentioned "lower alkyl" substituted with one or more the above mentioned "heteroaryl" at any possible position. Examples of the heteroarylalkyl are thienylmethyl (e.g., 2- thienylmethyl), thienylethyl (e.g., 2-(thiophen-2-yl)ethyl), furylmethyl (e.g., 2- furylmethyl), furylethyl (e.g., 2-(furan-2-yl)ethyl), pyrrolylmethyl (e.g., 2-pyrrolylmethyl), pyrrolylethyl (e.g., 2-(pyrrol-2-yl)ethyl), imidazolylmethyl (e.g., 2-imidazolylmethyl, 4-imidazolylmethyl), imidazolylethyl (e.g., 2-(imidazol-2-yl)ethyl), pyrazolylmethyl (e.g., 3- pyrazolylmethyl), pyrazolylethyl (e.g., 2-(pyrazol-3-yl)ethyl), thiazolylmethyl (e.g., 2-thiazolylmethyl), thiazolylethyl (e.g., 2-(thiazol-2-yl)ethyl), isothiazolylmethyl (e.g., 3-thiazolylmethyl), isoxazolylmethyl (e.g., 3-isoxazolylmethyl), oxazolylmethyl (e.g., 2-oxazolylmethyl), oxazolylethyl (e.g., 2-(oxazol-2-yl)ethyl), pyridylmethyl (e.g., 2-pyridylmethyl , 3-pyridylmethyl, 4-pyridylmethyl), pyridylethyl (e.g., 2-pyridylethyl) and the like.

The term "arylene" herein used means a divalent group of the above mentioned "aryl". Examples of the arylene include phenylene, naphthylene, and the like, in more detail, 1,2-phenylene, 1,3-phenylene, 1,4-phenylene, and the like. 1,4-Phenylene is preferred.

The term "heteroarylene" herein used means a divalent group of the above mentioned "heteroaryl". Example of the heteroarylene include thiofendiyl, furandiyl, pyridinediyl, and the like. In more detail, include 2,5-thiofendiyl, 2,5-furandiyl, 2,5-pyridinediyl and the like.

The term "non-aromatic heterocyclicdiyl" herein used means a divalent group of the above mentioned "non-aromatic heterocyclic group". Example of the non-aromatic heterocyclicdiyl include pyrrolidinediyl, piperidinediyl, pyrazinediyl, and the like.

The term "cycloalkylene" herein used means a divalent group of the above mentioned "cycloalkyl". Example of the cycloalkylene include cyclopentylene cyclohexylene, and the like.

The term "alkyloxy" herein used are methyloxy, ethyloxy, n-propyloxy, isopropyloxy, n-butyloxy, isobutyloxy, sec-butyloxy, tert-butyloxy, n-pentyloxy, n-hexyloxy, n-heptyloxy, n-octyloxy, n-nonanyloxy, n-decanyloxy, and the like. Methyloxy, ethyloxy, n-propyloxy, isopropyloxy and n-butyloxy are preferred.

The term "lower alkyloxy" herein used are methyloxy, ethyloxy, n-propyloxy, isopropyloxy, n-butyloxy, isobutyloxy, sec-butyloxy, tert-butyloxy, and the like. Methyloxy, ethyloxy, n-propyloxy, isopropyloxy and n-butyloxy are preferred.

The term "lower alkylthio" herein used are methylthio, ethylthio, and the like.

The term "lower alkyloxycarbonyl" herein used are methyloxycarbonyl, ethyloxycarbonyl, n-propyloxycarbonyl, isopropyloxycarbonyl, n-butyloxycarbonyl, t-butyloxycarbonyl, n-pentyloxycarbonyl and the like.

The term "aryloxycarbonyl" herein used are phenyloxycarbonyl, 1-naphthyloxycarbonyl, 2-naphthyloxycarbonyl, and the like.

In the present specification, the term "acyl" employed alone or in combination with other terms means alkylcarbonyl in which alkyl group is the above mentioned "lower alkyl" and arylcarbonyl in which aryl group is the above mentioned "aryl". Examples of the acyl are acetyl, propyonyl, benzoyl, and the like. "Lower alkyl" and "aryl" may be substituted respectively with substituents mentioned below.

In the present specification, the term "halo(lower)alkyl" employed alone or in combination with other terms means the above mentioned "lower alkyl" which is substituted with the above mentioned "halogen" at 1 to 8 positions, preferably, at 1 to 5 . Examples of the halo(lower)alkyl include trifluoromethyl, trichloromethyl, difluoroethyl, trifluoroethyl, dichloroethyl, trichloroethyl, and the like. Preferable is trifluoromethyl.

The term "halo(lower)alkyloxy" herein used are trifluoromethyloxy, trichloromethyloxy, difluoroethyloxy, trifluoroethyloxy, dichloroethyloxy, trichloroethyloxy, and the like. Preferable is trifluoromethyloxy.

Examples of the term "acyloxy" herein used are acetyloxy, propionyloxy, benzoyloxy and the like.

Examples of the term "lower alkylsilyl" herein used are triethylsilyl, t-butyldimethylsilyl, and the like.

In the present specification, the term "optionally substituted amino" employed alone or in combination with other terms includes amino substituted with one or two of the above mentioned "lower alkyl", "aryl", "aralkyl", "heteroaryl", "heteroarylalkyl" or "acyl". Examples of the optionally substituted amino include amino, methylamino, dimethylamino, ethylmethylamino, diethylamino, benzylamino, acetylamino, benzoylamino and the like. Preferable are amino, methylamino, dimethylamino, ethylmethylamino, diethylamino and acetylamino.

Examples of the term "optionally substituted aminocarbonyl" herein used are aminocarbonyl, methylaminocarbonyl, dimethylaminocarbonyl, ethylmethylaminocarbonyl, diethylaminocarbonyl and the like. Preferable are aminocarbonyl, methylaminocarbonyl, and dimethylaminocarbonyl.

In the present specification, the term "optionally substituted ureide" includes ureide substituted with one or more of the above mentioned "lower alkyl", "aryl", "aralkyl", "heteroaryl", "heteroarylalkyl" or "acyl".

The substituents of "optionally substituted lower alkyl" include cycloalkyl, lower alkenyl, lower alkyliden (e.g., ethylidene, propylidene), hydroxy, lower alkyloxy, mercapto, lower alkylthio, halogen, nitro, cyano, carboxy, lower alkyloxycarbonyl, halo(lower)alkyl, halo(lower)alkyloxy, optionally substituted amino, optionally substituted aminocarbonyl, acyl, acyloxy, optionally substituted non-aromatic heterocyclic group, aryloxy (e.g., phenyloxy), aralkyloxy (e.g., benzyloxy), lower alkylsulfonyl, guanidino, azo group, optionally substituted ureide, =N-O- (acyl) and the like. These substituents are able to locate at one or more of any possible positions.

Preferable are halogen atom, halo(lower)alkyl, and the like as substituents of "optionally substituted lower alkyl" for R^{C} and R^{D}.

Preferable are lower alkyloxycarbonyl and halogen atom as substituents of "optionally substituted lower alkyl" for R⁶, R⁷, and R⁹.

Preferable are cycloalkyl, lower alkenyl, lower alkylidene (e.g., ethylidene, propylidene, and the like), and the like as substituents of "optionally substituted lower alkyl" for R⁸.

The substituents of "optionally substituted lower alkyloxy" and "optionally substituted lower alkylthio" include cycloalkyl, lower alkenyl, lower alkylidene (e.g., ethylidene, propylidene, and the like), hydroxy, lower alkyloxy, mercapto, lower alkylthio, halogen, nitro, cyano, carboxy, lower alkyloxycarbonyl, halo(lower)alkyl, halo(lower)alkyloxy, optionally substituted amino, optionally substituted aminocarbonyl, acyl, acyloxy, optionally substituted non-aromatic heterocyclic group, aryloxy (e.g., phenyloxy), aralkyloxy (e.g., benzyloxy), lower alkylsulfonyl, guanidino, azo group, optionally substituted ureide, =N-O- (acyl) and the like. These substituents are able to locate at one or more of any possible positions.

The substituents of "optionally substituted lower alkenyl" and "optionally substituted lower alknyl" include cycloalkyl, lower alkenyl, lower alkylidene (e.g., ethylidene, propylidene, and the like), hydroxy, lower alkyloxy, mercapto, lower alkylthio, halogen, nitro, cyano, carboxy, lower alkyloxycarbonyl, halo(lower)alkyl, halo(lower)alkyloxy, optionally substituted amino, optionally substituted aminocarbonyl, acyl, acyloxy, optionally substituted non-aromatic heterocyclic group, aryloxy (e.g., phenyloxy), aralkyloxy (e.g., benzyloxy), lower alkylsulfonyl, guanidino, azo group, optionally substituted ureide, and the like. These substituents are able to locate at one or more of any possible positions.

The substituents of "optionally substituted arylene", "optionally substituted heteroarylene", "optionally substituted 2,5-pyridinediyl", "optionally substituted 2,5-thiophenediyl", "optionally substituted 2,5-furandiyl", "optionally substituted non-armatic heterocyclicdiyl", "optionally substituted cycloalkylene", "optionally substituted aryl", "optionally substituted phenyl", "optionally substituted heteroaryl", "optionally substituted non-aromatic heterocyclic group", "optionally substituted cycloalkyl", "optionally substituted aralkyl", and "optionally substituted heteroarylalkyl" herein used are optionally substituted alkyl, cycloalkyl, lower alkenyl, lower alkynyl, hydroxy, alkyloxy,aralkyloxy, mercapto, lower alkylthio, halogen, nitro, cyano, carboxy, lower alkyloxycarbonyl, halo(lower)alkyl, halo(lower)alkyloxy, optionally substituted amino, optionally substituted aminocarbonyl, acyl, acyloxy, optionally substituted aryl (which is substituted by halogen atom, carboxy, alkyl, or alkyloxy, and the like), optionally substituted heteroaryl (which is substituted by halogen atom, carboxy, alkyl, or alkyloxy, and the like), optionally substituted non-aromatic heterocyclic group, optionally substituted aralkyl, lower alkylsulfonyl, guanidino, azo group, -N=N-(optionally substituted phenyl) or optionally substituted ureide and the like. These substituents are able to locate at one or more of any possible positions.

In the present specification, the term "(α)_{β-γ"} means that the number of α present is β to γ. For examples, (CR^{C}R^{D})_{0-2,} (CH₂)_{0-2,} and (CH₂)₁₋₅ mean that CR^{C}R^{D} is present 0 to 2, CH₂ is present 0 to 2, CH₂ is present 1 to 5, respectively.

In the present specification, the term "platelet production modifier" includes a medicine for hemopathy accompanied with the unusual number of platelet. For example the hemopathy is thrombocytopenia (after bone marrow transplantation, after chemotherapy, anaplastic anemia, bone marrow dysplasia syndrome, acquired thrombopenia of intractable sudden thrombocy topenic purpura and the like, congenital thrombopenia of thrombopoietin deficiency and the like) and the like. For example this medicine can be used as a treating agent for decreace platelet number caused by administrating an antitumor agent, or as a preventing agent for the platelet number decreace caused by administrating an antitumor agent.

In the present specification, the term "modifying platelet production" includes 1) increasing the number of platelet decreased by administrating an antitumor agent and the like, 2) maintaining the number of platelet which may be decreased by administrating an antitumor agent and the like, and 3) reducing the ratio of the platelet number decrease caused by administrating an antitumor agent and the like.

The pharmaceuticl composition exhibiting thrombopoietin receptor agonism includes a thrombopoietin receptor agonistic agent.

### Best Mode for Carrying Out the Invention

Compounds (I) of the invention can be synthesized by the following methods A to C and the similar process. Furthermore, they can be synthesized in a manner similar to the methods described in WO97/05135 and WO98/39737.

### (Method A)

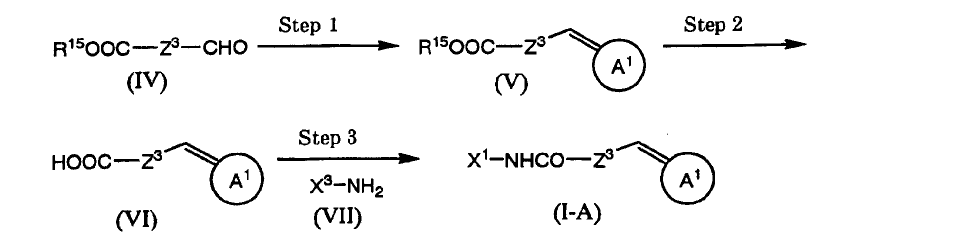

wherein A¹ and X¹ are as defined above; Z³ is optionally substituted arylene, optionally substituted heteroarylene, optionally substituted non-aromatic heterocyclicdiyl, or optionally substituted cycloalkylene; R¹⁵ is lower alkyl.

### (Step 1)

Commercial available compounds (IV) substituted with lower alkyloxycarbonyl and aldehyde are used as a starting material.

Further, the compounds (IV) can be obtained by the following methods 1) to 3), 1) the carboxy group of the compounds substituted with lower alkyloxycarbonyl and carboxy is converted to mixed acid anhydride with ethyl chlorocarbonate or the like, 2) the obtained compounds are converted to the compounds substituted with alkyloxycarbonyl and hydroxy by usual reduction reaction (e.g., reduction reaction with sodium borohydride), 3) the obtained compounds are converted to the compounds (IV) substituted with alkyloxycarbonyl and aldehyde by usual oxidation reaction (e.g., Swern oxidation, Dess-Martin oxidation, or the like).

This step is a process for preparing benzylidene derivatives by reacting aldehyde derivatives (IV) and 2,4-thiazolidinedione or the like. The desired compounds can be obtained by reacting the compounds (IV) in a solvent such as benzene, toluene with 2,4-thiazolidinedione or the like under heating and reflux in the presence of acetic acid and piperidine as catalyst (Knoevenagel reaction).

The produced double bond can be reduced by usual reduction reaction (e.g., catalytic reduction) at appropriate stage. When a substituent interfering with the reaction is present, it can be protected by the method described in Protective Groups in Organic Synthesis, Theodora W Green (Johan Wiley & Sons) or the like, and deprotected at appropriate stage.

### (Step 2)

This step is a process for converting alkyloxy derivatives to carboxylic acid derivatives by hydrolysis. It can be conducted on usual hydrolysis reaction. For example, carboxylic derivatives (compound (VI)) can be obtained by reacting the compounds (V) in acetic acid with hydrochloric acid or the like.

### (Step 3)

This step is a process for preparing amide derivative (I-A) from carboxylic acid derivatives (VI) and amine derivatives (VII) by the method such as active esterification, acid chloride, and mixed acid anhydride. This step is reacted in a solvent such as tetrahydrofuran, dioxane, dichloromethane, toluene, and benzene. The active esterification can be carried out by using 1-hydroxybenzotriazole, hydroxysuccinimide, dimethylaminopyridine, and the like and a condensation reagent such as dicyclohexylcarbodiimide and 1-ethyl-3-(3-dimethylaminopropyl)carbodiimide hydrochloride salt. The acid halide method can be carried out by converting free carboxylic acid to acid chloride with thionyl chloride or oxalyl chloride. The mixed acid anhydride method can be carried out by converting carboxylic acid to mixed acid anhydride with ethylchloroformate, isobutylchloroformate or the like. Triethylamine, pyridine or the like are used as base in these reaction if necessary.

Compounds (VII) can be commercially available or obtained by the following methods. 1) in the case that X¹ is optionally substituted aryl, optionally substituted heteroaryl or the like, and said substituent is aryl and heteroaryl, compounds (VII) having a continuously connected two rings can be obtained by Suzuki reaction or the like. 2) in the case that X¹ is optionally substituted thiazole, compounds (VII') can be obtained by the following method. 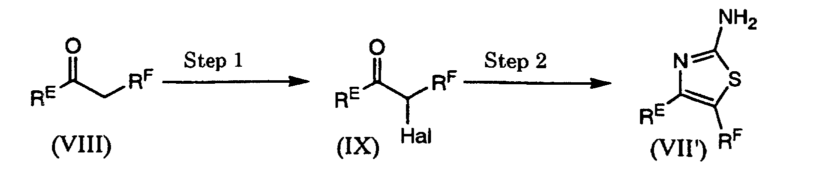 wherein R^{E} and R^{F} are hydrogen atom, optionally substituted lower alkyl, carboxy, lower alkyloxycarbonyl, optionally substituted aminocarbonyl, optionally substituted phenyl, or the like; Hal is halogen atom.

### (Step 1)

This step is a process of halogenation. It can be prepared by usual halogenation. For example, it can be brominated by reacting with bromine in a mixed solvent of methanol-chloroform.

### (Step 2)

This step is a process for constructing thiazole ring. For example, the desired thiazole derivatives (VII') can be obtained by reacting with thiourea in a solvent such as methanol.

### (Method B)

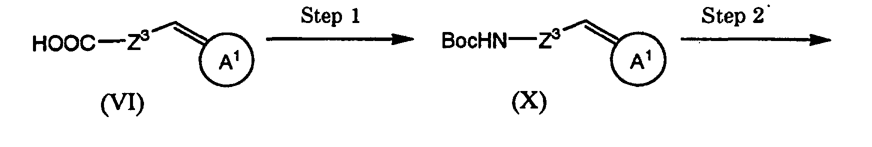

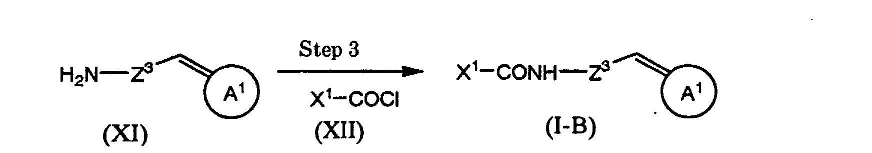

wherein A¹, X¹, and Z³ are as defined above; Boc is t-butyloxycarbonyl.

### (Step 1)

This step is a process for converting carboxy to amino protected with Boc. For example, the desired compounds can be obtained by reacting the compounds (VI) having carboxy in a solvent such as dimethylformamide, toluene, diethyl ether, dioxane, with t-butanol and diphenylphosphorylazide, in the presence of a base such as triethylamine .

### (Step 2)

This step is a process for removing Boc. It can be conducted by the method described in Protective Groups in Organic Synthesis, Theodora W Green (Johan Wiley & Sons) or the like. For example, the desired deprotected derivatives (XI) can be obtained by treating the compounds (X) with trifluoroacetic acid.

### (Step 3)

It can be conducted in a manner similar to Step 3 of Method A.

### (Method C)

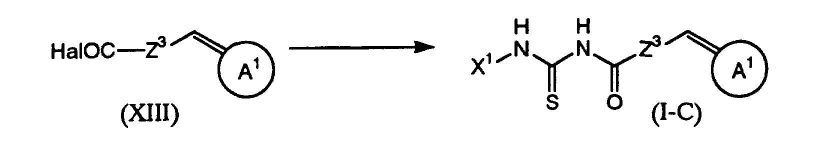

wherein A¹, X¹, Z³, and Hal are as defined above.

This step is a process for converting the carboxylic acid halide (XIII) of the compounds (VI) described in Method A to the desired compounds (I-C) by treating with ammonium isothiocyanate, followed by reacting with the above mentioned compounds (VII).

The compounds of the general formula (I) wherein Y¹ is not -CONH-, -NHCO-, and -NHC(=S)NHC(=O)- can be synthesized in a manner similar to Method A to C described above.

N-alkyl derivatives can be prepared by usual alkylation.

In the synthesis of a compound (I) wherein Y¹ is -N(-alkyl)-CO-; X¹ is optionally substituted thiazole, represented by the formula; 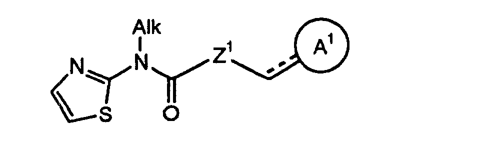 wherein A¹ and Z¹ are as defined above; Alk is lower alkyl, depending the alkylation condition, the following compound may be obtained. 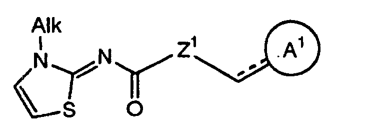 wherein A¹, Z¹ and Alk are as defined above.

In the case of compound of the general formula (I), (II), or (III) wherein a broken line represents the presence of a bond, the compound includes a cis isomer and a trans isomer. For example, in the case that A¹ ring is thiazolidinedione, the following cis isomer and trans isomer may exist. 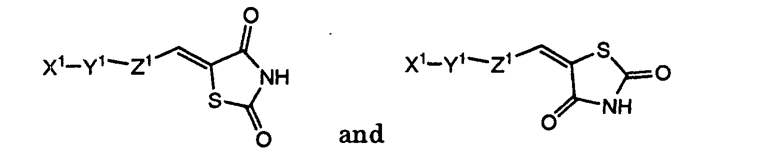 wherein X¹, Y¹ and Z¹ are as defined above.

The term "solvate" includes, for example, solvates with organic solvents, hydrates, and the like.

The term "compound of the present invention" herein used includes a pharmaceutically acceptable salt or hydrate thereof. The salt is exemplified by a salt with alkali metals (e.g., lithium, sodium, potassium, and the like), alkaline earth metals (e.g., magnesium, calcium, and the like), ammonium, organic bases, amino acids, mineral acids (e.g., hydrochloric acid, hydrobromic acid, phosphoric acid, sulfuric acid, and the like), or organic acids (e.g., acetic acid, citric acid, maleic acid, fumaric acid, benzenesulfonic acid, p-toluenesulfonic acid, and the like). These salts can be formed by the usual method. These hydrates can coordinate with any water molecules.

Prodrug is a derivative of the compound having a group which can be decomposed chemically or metabolically, and such prodrug is a compound according to the present invention which becOMes pharmaceutically active by means of solvolysis or by placing the compound in vivo under a physiological condition. The method of both selection and manufacture of appropriate prodrug derivatives is described in, for example. Design of Prodrugs, Elsevier, Amsterdam, 1985). For instance, prodrugs such as an ester derivative which is prepared by reacting a basal acid compound with a suitable alcohol, or an amide derivative which is prepared by reacting a basal acid compound with a suitable amine are exemplified when the compounds according to present invention have a carboxylic group. Particularly preferred esters as prodrugs are methyl, ethyl, n-propyl, isopropyl, n-butyl, isobutyl, tert-butyl, morpholinoethyl, and N,N-diethylglycolamido, and the like. For instance, prodrugs such as an acyloxy derivative which is prepared by reacting a basal hydroxy compound with a suitable acyl halide or a suitable acid anhydride, or an amide derivative which is prepared by reacting a basal acid compound with a suitable amine are exemplified when the compounds according to present invention have a hydroxy group.. Particularly preferred acyloxy derivatives as prodrugs -OCOC₂H₅, -OCO(t-Bu), -OCOC₁₅H_{31,} -OCO(m-COONa-Ph),-COCH₂CH₂COONa, -OCOCH(NH₂)CH₃, -OCOCH₂N(CH₃)₂, and the like. For instance, prodrugs such as an amide derivative which is prepared by reacting a basal amino compound with a suitable acid halide or a suitable acid anhydride are exemplified when the compounds according to present invention have an amino group. Particularly preferred amide as prodrugs are -NHCO(CH₂)₂₀CH₃, -NHCOCH(NH₂)CH₃, and the like.

The compound of the present invention is not restricted to any particular isomers but includes all possible isomers and racemic modifications.

The present invention compounds show excellent thrombopoietin receptor agonism as described in examples mentioned later, and may be used as a pharmaceutical composition (platelet production modifier) for hemopathy accompanied with the unusual number of platelet. And the present compounds may be used as a peripheral blood stem cell-releasing accelerating agent, a differetiation-inducing agent against megakaryocytoid leukemia cell, a thrombocytosis agent for blood platelet donor, and the like.

When the compound of the present invention is administered to a person for the treatment of the above diseases, it can be administered orally as powder, granules, tablets, capsules, pilulae, and liquid medicines, or parenterally as injections, suppositories, percutaneous formulations, insufflation, or the like. An effective dose of the compound is formulated by being mixed with appropriate medicinal admixtures such as excipient, binder, penetrant, disintegrators, lubricant, and the like ifnecessary. Parenteral injections are prepared by sterilizing the compound together with an appropriate carrier.

The dosage varies with the conditions of the patients, administration route, their age, and body weight. In the case of oral administration, the dosage can generally be between 0. 1 to 100 mg/kg/day, and preferably 1 to 20 mg/kg/day for adult.

The following examples are provided to further illustrate the present invention and are not to be construed as limiting the scope thereof.

Abbreviations described below are used in the following examples.
Me : methyl
Et : ethyl
n-Pr : n-propyl
i-Pr : isopropyl
c-Pr: cyclopropyl
n-Bu: n-butyl
i-Bu : isobutyl
sec-Bu : sec-butyl
t-Bu : tert-butyl
i-Bu : isobutyl
n-Pen : n-pentyl
c-Pen : cyclopentyl
n-Hex: n-hexyl
c-Hex : cyclohexyl
i-Hex: isohexyl
Ph : phenyl
Bn : benzyl
Bz : benzoyl
Py : pyridyl
Th thienyl
Ac : acetyl
Z: benzyloxycarbonyl
DMF : N,N-dimethylformamide
THF : tetrahydrofuran

### Example

### Example 1 Preparation of compound (A-1)

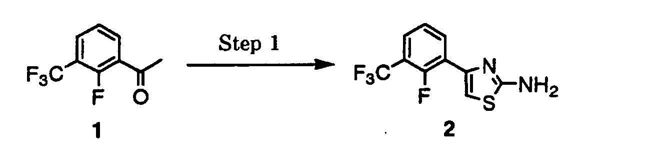

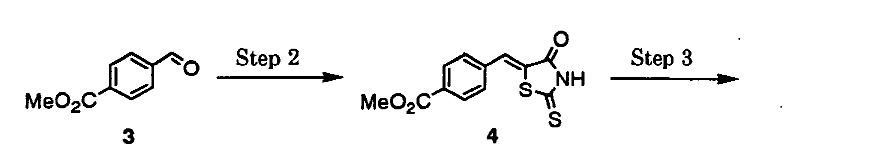

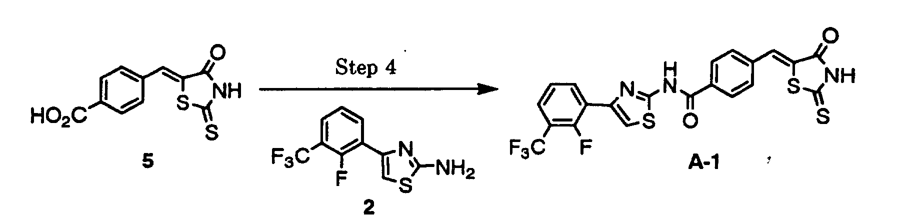

### (Step 1)

To a solution of 2'-fluoro-3'-trifluoroacetophenone (1) (20.9 g) in 10% methanol-chloroform was added bromine (5.26 mL), and the reaction mixture was stirred at room temperature until a color of bromine was disappeared. The solvent was evaporated under reduced pressure and the residue was dissolved in ethanol again. To the reaction mixture was added thiourea (7.71 g) and the mixture was heated under reflux for 2 h. To the reaction mixture was added a mixture of ethyl acetate-saturated aqueous sodium bicarbonate solution, and the organic layer was dried over anhydrous magnesium sulfate, and evaporated. The residue was purified by column chromatography to give a compound (2) (19.8 g).
¹H NMR (CDCl₃, δ ppm): 8.22 - 8.28 (m, 1H), 7.48 - 7.54 (m, 1H), 7.24 - 7.30 (m, 1H), 7.10 (d, 1H, J = 2.4 Hz), 5.14 (bs, 2H).

### (Step 2)

To a solution of methyl terephthalaldehydate (25 g) and rhodanine (23.3 g) in toluene were added 1 mol/L piperizine-toluene solution (6.2 mL) and 1 mol/L acetic acid-toluene solution (6.2 mL), and then the reaction mixture was heated under reflux overnight. After cooling, the resulting crystal was filtered to a compound (4) (34.6 g) as a brown oil.
¹H NMR(DMSO-d₆, δ ppm) 13.18 (bs, 1H), 8.07 (d, 2H, J = 8.7 Hz), 7.73 (d, 2H, J = 8.7 Hz), 7.68 (s, 1H), 3.88 (s, 1H).

### (Step 3)

A suspension of compound (4) (34.6 g) in dioxane (160 mL), acetic acid (250 mL), and 6 mol/L hydrochloric acid (88 mL) was refluxed at 120 °C for 5 h. To the reaction mixture was added water (350 mL), after cooling, the crystal was filtered to give a compound (5) (30.0 g).
¹H NMR (DMSO-d₆, δ ppm): 13.95 (bs, 1H), 13.24 (bs, 1H), 8.06 (d, 2H, J = 8.4 Hz), 7.72 (d, 2H, J = 8.4 Hz), 7.69 (s, 1H).

### (Step 4)

A mixture of compound (5) (3 g) in dioxane (20 mL) and thionyl chloride (10 mL) was heated to dissolve at 100 °C. The solvent was evaporated to give a carboxylic acid chloride. The obtained carboxylic acid chloride was used at next reaction without purification. The carboxylic acid chloride (286 mg) and the compound (2) (368 mg) synthesized in Step 1 were dissolved in dioxane (50 mL), and pyridine (162 µL) was added to the reaction mixture and the reaction mixture was heated at 100 °C for 2 h. After cooling, the solvent was evaporated, and to the residue were added methanol (6 mL) and water (2 mL). The resulting crystal was filtered and recrystallized from DMF to give a compound (A-1) (220 mg).
¹H NMR (DMSO-d₆, δ ppm): 13.90 (bs, 1H), 13.02 (bs, 1H), 8.37 - 8.42 (m, 1H), 8.25 (d, 2H, J = 8.2 Hz), 7.75 - 7.81 (m, 4H), 7.71 (s, 1H), 7.53 - 7.58 (m, 1H).

Compounds (A-2) to (A-51) were synthesized in a manner similar to Example 1. Their physical data were shown in Tables 1 to 6.

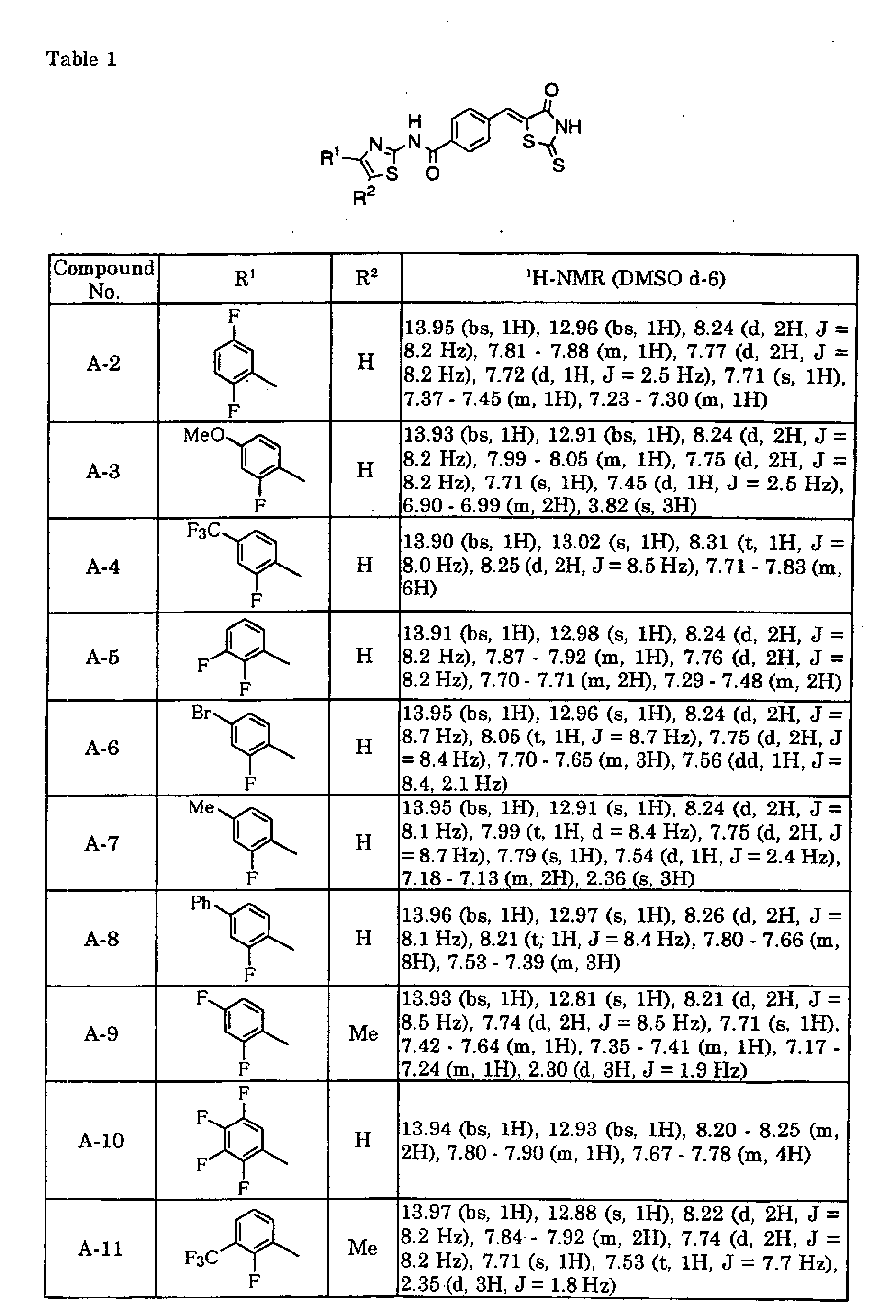

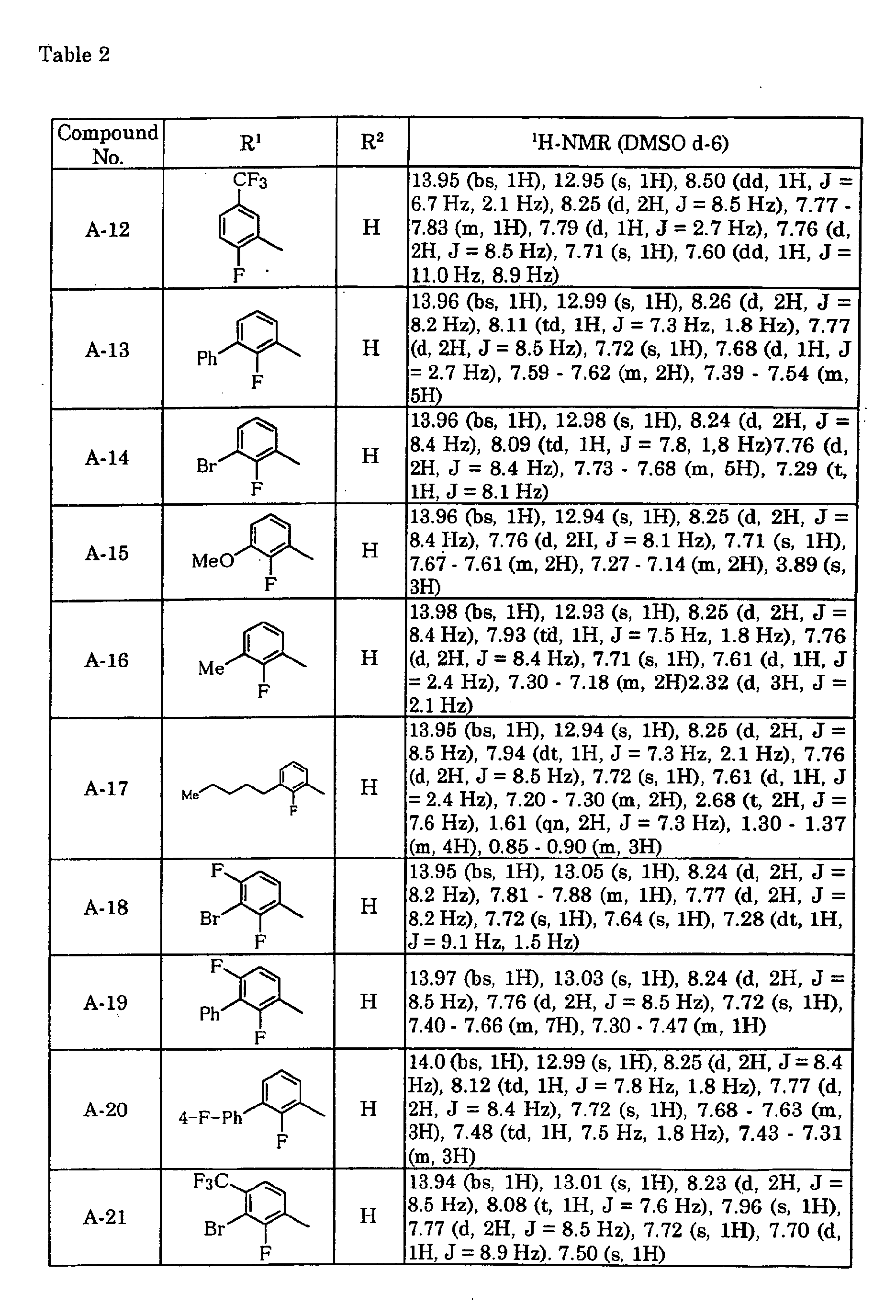

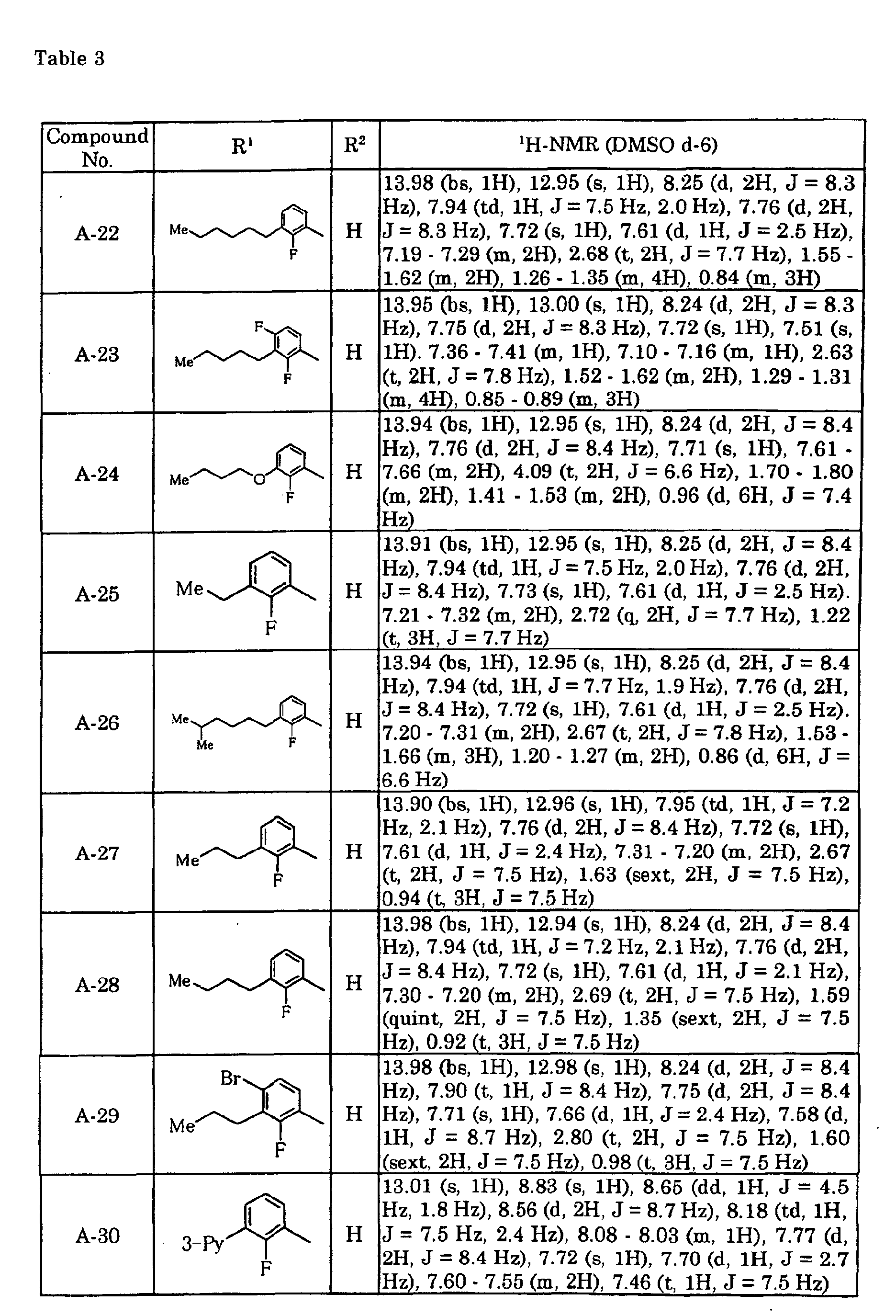

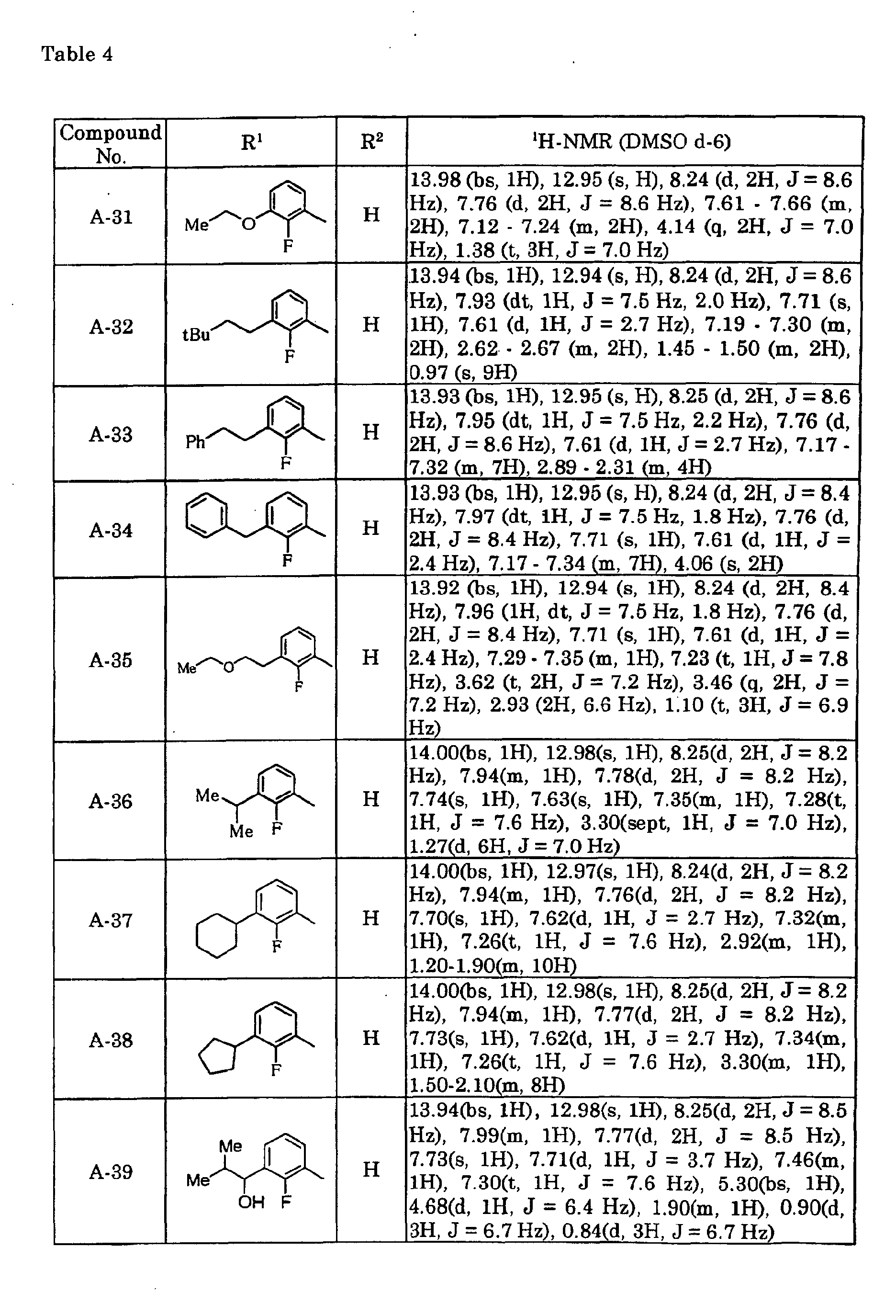

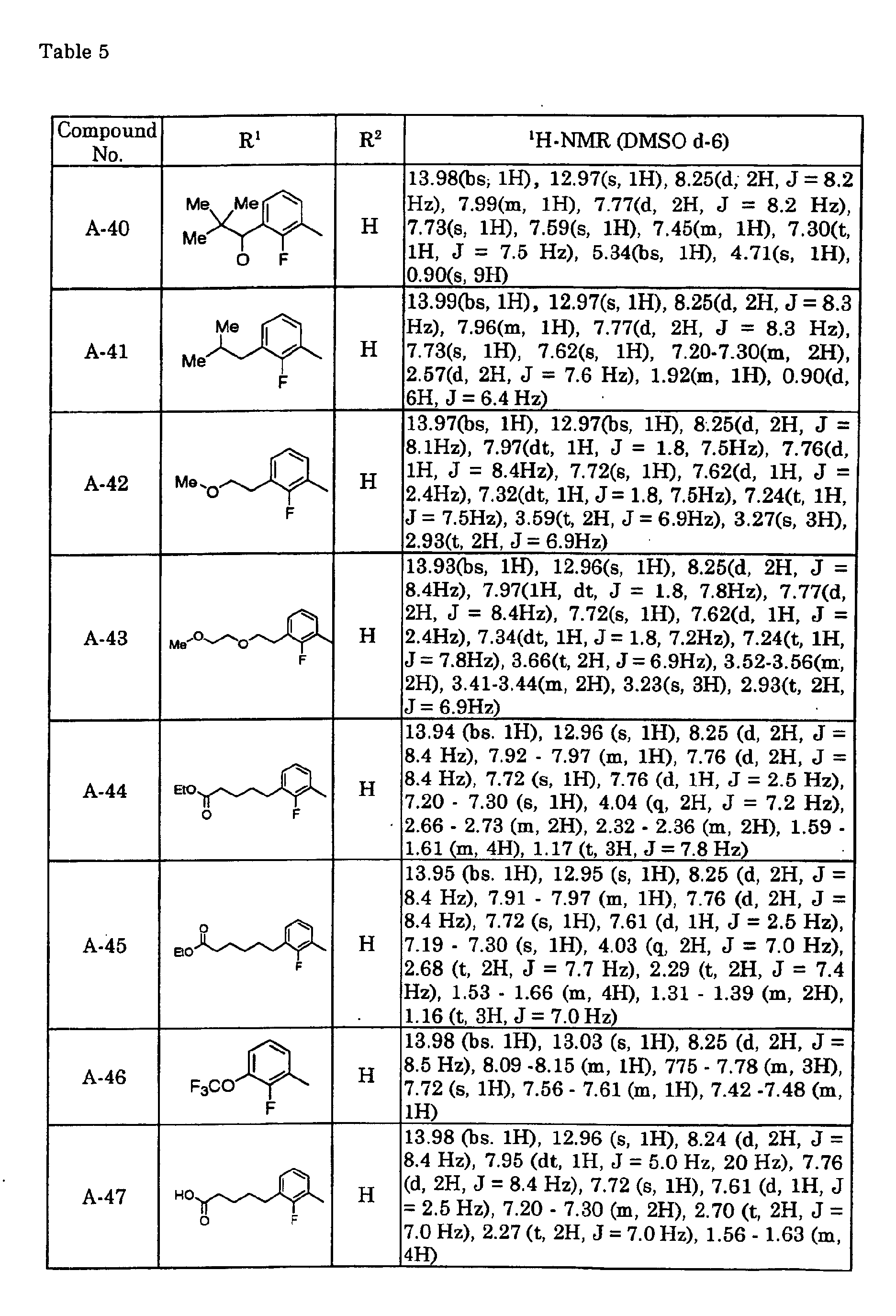

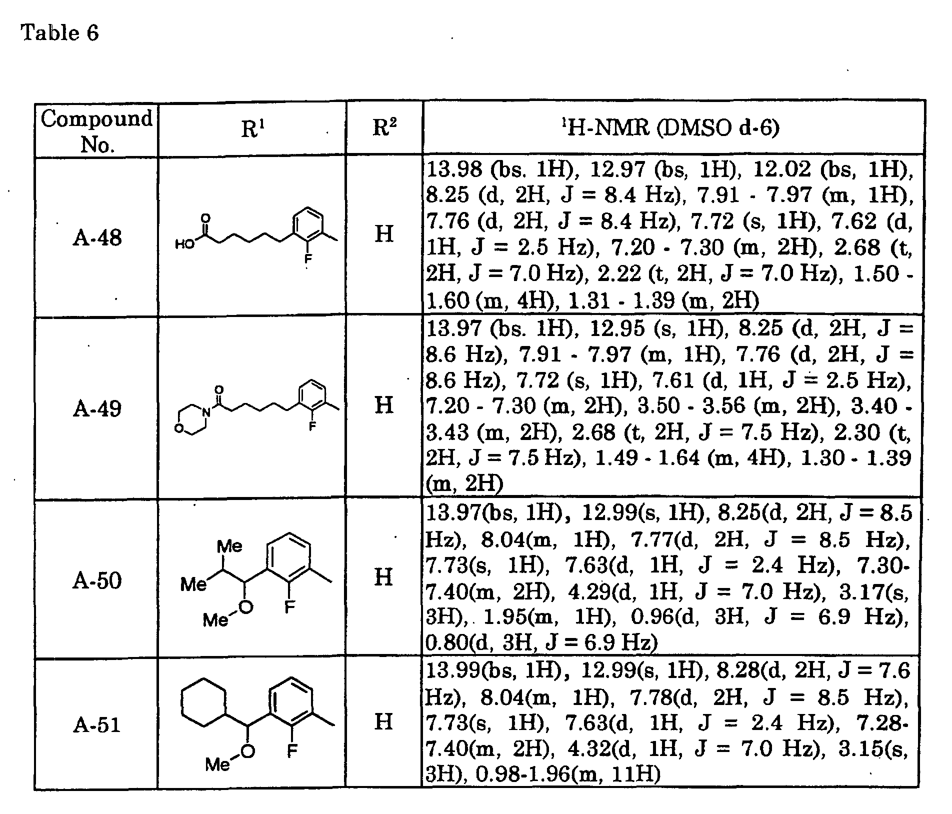

All compounds of the general formula (XIV) and (XV), having substituent of the following combination, can be synthesized in a manner similar to the method described above. 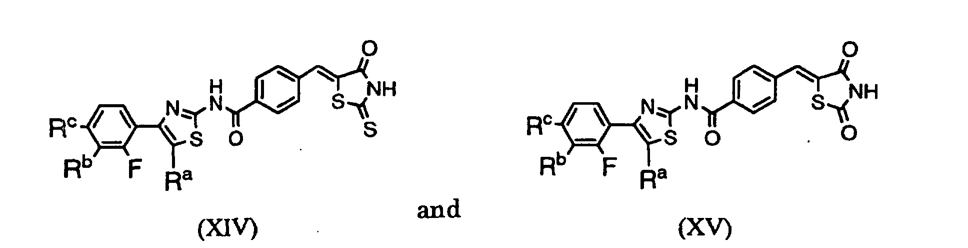 wherein R^{a} is hydrogen atom, fluoro, or methyl; R^{b} is hydrogen atom, fluoro, or chloro; R^{c} is hydrogen atom, fluoro, chloro, methyl, ethyl, n-propyl, cyclopropyl, isopropyl, n-butyl, isobutyl, sec-butyl, n-pentyl, cyclopentyl, n-hexyl, cyclohexyl, hydroxy, methyloxy, ethyloxy, n-propyloxy, phenyloxy, benzyloxy, phenylethyloxy, trifluoromethyl, trifluoromethyloxy, phenyl, 4-fluorophenyl, 4-trifluoromethylphenyl, 4-dimethylaminophenyl, 4-hydroxyphenyl, 3,4-difluorophenyl, 4-carboxyphenyl, benzyl, 4-fluorobenzyl, 2-pyridyl, 3-pyridyl, 4-pyridyl, 2-thienyl, 3-thienyl, pyrazol-2-yl, pyrazol-3-yl, pyrimidin-2-yl, pyrimidin-4-yl, pyrimidin-5-yl, 3-carboxypropyl, 4-carboxybutyl, 4-dimethylaminocarbonylbutyl, 5-dimethylaminocarbonylpentyl, methyloxymethyl, ethyloxymethyl, ethyloxyethyl, methyloxyethyloxyethyl, methyloxyethyl, hydroxymethyl, hydroxyethyl, hydroxypropyl, hydroxybutyl, hydroxypentyl, dimethylamino, piperidin-4-ylmethyl, or cyclohexylmethyl; R^{c} is hydrogen atom, fluoro, chloro, bromo, methyl, or trifluoromethyl;
(R^{a}, R^{b}, R^{c}) = (B-1, H, H, H), (B-2, H, H, Cl), (B-3, H, H, F), (B-4, H, H, CF₃), (B-5, H, H, Br), (B-6, H, H, Me), (B-7, H, F, H), (B-8, H, F, Cl), (B-9, H, F, F), (B-10, H, F, CF₃), (B-11, H, F, Br), (B-12, H, F, Me), (B-13, H, Cl, H), (B-14, H, Cl, Cl), (B-15, H, Cl, F), (B-16, H, Cl, CF₃), (B-17, H, Cl, Br), (B-18, H, Cl, Me), (B-19, H, Me, H), (B-20, H, Me, Cl), (B-21, H, Me, F), (B-22, H, Me, CF₃), (B-23, H, Me, Br), (B-24, H, Me, Me), (B-25, H, Et, H), (B-26, H, Et, Cl), (B-27, H, Et, F), (B-28, H, Et, CF₃), (B-29, H, Et, Br), (B-30, H, Et, Me), (B-31, H, n-Pr, H), (B-32, H, n-Pr, Cl), (B-33, H, n-Pr, F), (B-34, H, n-Pr, CF₃), (B-35, H, n-Pr, Br), (B-36, H, n-Pr, Me), (B-37, H, c-Pr, H), (B-38, H, c-Pr, Cl), (B-39, H, c-Pr, F), (B-40, H, c-Pr, CF₃), (B-41, H, c-Pr, Br), (B-42, H, c-Pr, Me), (B-43, H, i-Pr, H), (B-44, H, i-Pr, Cl), (B-45, H, i-Pr, F), (B-46, H, i-Pr, CF₃), (B-47, H, i-Pr, Br), (B-48, H, i-Pr, Me), (B-49, H, n-Bu, H), (B-50, H, n-Bu, Cl), (B-51, H, n-Bu, F), (B-52, H, n-Bu, CF₃), (B-53, H, n-Bu, Br), (B-54, H, n-Bu, Me), (B-55, H, i-Bu, H), (B.56, H, i-Bu, Cl), (B-57, H, i-Bu, F), (B-58, H, i-Bu, CF₃), (B-59, H, i-Bu, Br), (B-60, H, i-Bu, Me), (B-61, H, sec-Bu, H), (B-62, H, sec-Bu, Cl), (B-63, H, sec-Bu, F), (B-64, H, sec-Bu, CF₃), (B-65, H, sec-Bu, Br), (B-66, H, sec-Bu, Me), (B-67, H, n-Pen, H), (B-68, H, n-Pen, Cl), (B-69, H, n-Pen, F), (B-70, H, n.Pen, CF₃), (B-71, H, n-Pen, Br), (B-72, H, n-Pen, Me), (B-73, H, c-Pen, H), (B-74, H, c-Pen, Cl), (B-75, H, c-Pen, F), (B-76, H, c-Pen, CF₃), (B-77, H, c-Pen, Br), (B-78, H, c-Pen, Me), (B-79, H, n-Hex, H), (B-80, H, n-Hex, Cl), (B-81, H, n-Hex, F), (B-82, H, n-Hex, CF₃), (B-83, H, n-Hex, Br), (B-84, H, n-Hex, Me), (B-85, H, c-Hex, H), (B-86, H, c-Hex, Cl), (B-87, H, c-Hex, F), (B-88, H, c-Hex, CF₃), (B-89, H, c-Hex, Br), (B-90, H, c-Hex, Me), (B-91, H, OH, H), (B-92, H, OH, Cl), (B-93, H, OH, F), (B-94, H, OH, CF₃), (B-95, H, OH, Br), (B.96. H, OH, Me), (B-97, H, MeO, H), (B-98, H, MeO, Cl), (B-99, H, MeO, F), (B-100, H, MeO, CF₃), (B-101, H, MeO, Br), (B-102, H, MeO, Me), (B-103, H, EtO, H), (B-104, H, EtO, Cl), (B-105, H, EtO, F), (B-106, H, EtO, CF₃), (B-107, H, EtO, Br), (B-108, H, EtO, Me), (B-109, H, n-PrO, H), (B-110, H, n-PrO, Cl), (B-111, H, n-PrO, F), (B-112, H, n-PrO, CF₃), (B-113, H, n-PrO, Br), (B-114, H, n-PrO, Me), (B-115, H, PhO, H), (B-116, H, PhO, Cl), (B-117, H, PhO, F), (B-118, H, PhO, CF₃), (B-119, H, PhO, Br), (B-120, H, PhO, Me), (B-121, H, BnO, H), (B-122, H, BnO, Cl), (B-123, H, BnO, F), (B-124, H, BnO, CF₃), (B-125, H, BnO, Br), (B-126, H, BnO, Me), (B-127, H, PhCH₂CH₂O, H), (B-128, H, PhCH₂CH₂O, Cl), (B.129, H, PhCH₂CH₂O, F), (B-130, H, PhCH₂CH₂O, CF₃), (B-131, H, PhCH₂CH₂O, Br), (B-132, H, PhCH₂CH₂O, Me), (B-133, H, CF₃, H), (B-134, H, CF_{3,} Cl)_{,} (B-135, H, CF₃, F), (B-136, H, CF₃, CF₃), (B-137, H, CF₃, Br), (B-138, H, CF₃, Me), (B-139, H, CF₃O, H), (B-140, H, CF₃O, Cl), (B-141, H, CF₃O, F), (B-142, H, CF₃O, CF₃), (B-143, H, CF₃O, Br), (B-144, H, CF₃O, Me), (B-145, H, Ph, H), (B-146, H, Ph, Cl), (B-147, H, Ph, F), (B-148, H, Ph, CF₃, (B-149, H, Ph, Br), (B-150, H, Ph, Me), (B-151, H, 4-F-Ph, H), (B-152, H, 4-F-Ph, Cl), (B-153, H, 4-F-Ph, F), (B-154, H, 4-F-Ph, CF₃), (B-155, H, 4-F-Ph, Br), (B-156, H, 4-F-Ph, Me), (B-157, H, 4-CF₃-Ph, H), (B-158, H, 4-CF₃-Ph, Cl), (B-159, H, 4-CF₃-Ph, F), (B-160, H, 4-CF₃-Ph, CF₃), (B-161, H, 4-CF₃-Ph, Br), (B-162, H, 4-CF₃-Ph, Me), (B-163, H, 4-(Me)₂N-Ph, H), (B-164, H, 4-(Me)₂N-Ph, Cl), (B-165, H, 4-(Me)₂N-Ph, F), (B-166, H, 4-(Me)₂N-Ph, CF₃), (B-167, H, 4-(Me)₂N-Ph, Br), (B-168, H, 4-(Me)₂N-Ph, Me), (B-169, H, 4-OH-Ph, H), (B-170, H, 4-OH-Ph, Cl), (B-171, H, 4-OH-Ph, F), (B-172, H, 4-OH-Ph, CF₃), (B-173, H, 4-OH-Ph, Br), (B-174, H, 4-OH-Ph, Me), (B-175, H, 3,4-di-F-Ph, H), (B-176, H, 3,4-di-F-Ph, Cl), (B-177, H, 3,4-di-F-Ph, F), (B-178, H, 3,4-di-F-Ph, CF₃), (B-179, H, 3,4-di-F-Ph, Br), (B-180, H, 3,4-di-F-Ph, Me), (B-181, H, 4-COOH-Ph, H), (B-182, H, 4-COOH-Ph, Cl), (B-183, H, 4-COOH-Ph, F), (B-184, H, 4-COOH-Ph, CF₃), (B-185, H, 4-COOH-Ph, Br), (B-186, H, 4-COOH-Ph, Me), (B-187, H, Bn, H), (B-188, H, Bn, Cl), (B-189, H, Bn, F), (B-190, H, Bn, CF₃), (B-191, H, Bn, Br), (8-192, H, Bn, Me), (B-193, H, 4-F-Bn, H), (B-194, H, 4-F-Bn, Cl), (B-195, H, 4-F-Bn, F), (B-196, H, 4-F-Bn, CF₃), (B-197, H, 4-F-Bn, Br), (B-198, H, 4-F-Bn, Me), (B-199, H, 2-Py, H), (B-200, H, 2-Py, Cl), (B-201, H, 2-Py, F), (B-202, H, 2-Py, CF₃), (B-203, H, 2-Py, Br), (B-204, H, 2-Py, Me), (B-205, H, 3-Py, H), (B-206, H, 3-Py, Cl), (B-207, H, 3-Py, F), (B-208, H, 3-Py, CF₃), (B-209, H, 3-Py, Br), (B-210, H, 3-Py, Me), (B-211, H, 4-Py, H), (B-212, H, 4-Py, Cl), (B-213, H, 4-Py, F), (B-214, H, 4-Py, CF₃), (B-215, H, 4-Py, Br), (B-216, H, 4-Py, Me), (B-217, H, 2-Th, H), (B-218, H, 2-Th, Cl), (B-219, H, 2-Th, F), (B-220, H, 2-Th, CF₃), (B-221, H, 2-Th, Br), (B-222, H, 2-Th, Me), (B-223, H, 3-Th, H), (B-224, H, 3-Th, Cl), (B-225, H, 3-Th, F), (B-226, H, 3-Th, CF₃), (B-227, H, 3-Th, Br), (B-228, H, 3-Th, Me), (B-229, H, Pyrazol-2-yl, H), (B-230, H, Pyrazol-2-yl, Cl), (B-231, H, Pyrazol-2-yl, F), (B-232, H, Pyrazol-2-yl, CF₃), (B-233, H, Pyrazol-2-yl, Br), (B-234, H, Pyrazol-2-yl, Me), (B-235, H, Pyrazol-3-yl, H), (B-236, H, Pyrazol-3-yl, Cl), (B-237, H, Pyrazol-3-yl, F), (B-238, H, Pyrazol-3-yl, CF₃), (B-239, H, Pyrazol-3-yl, Br), (B-240, H, Pyrazol-3-yl, Me), (B-241, H, pyrimidin-2-yl, H), (B-242, H, pyrimidin-2-yl, Cl), (B-243, H, pyrimidin-2-yl, F), (B-244, H, pyrimidin-2-yl, CF₃), (B-245, H, pyrimidin-2-yl, Br), (B-246, H, pyrimidin-2-yl, Me), (B-247, H, pyrimidin-4-yl, H), (B-248, H, pyrimidin-4-yl, Cl), (B-249, H, pyrimidin-4-yl, F), (B-250, H, pyrimidin-4-yl, CF₃), (B-251, H, pyrimidin-4-yl, Br), (B-252, H, pyrimidin-4-yl, Me), (B-253, H, pyrimidin-5-yl, H), (B-254, H, pyrimidin-5-yl, Cl), (B-255, H, pyrimidin-5-yl, F), (B-256, H, pyrimidin-5-yl, CF₃), (B-257, H, pyrimidin-5-yl, Br), (B-258, H, pyrimidin-5-yl, Me), (B-259, H, HOOCCH₂CH₂CH₂, H), (B-260, H, HOOCCH₂CH₂CH₂, Cl), (B-261, H, HOOCCH₂CH₂CH₂, F), (B-262, H, HOOCCH₂CH₂CH₂, CF₃), (B-263, H, HOOCCH₂CH₂CH₂, Br), (B-264, H, HOOCCH₂CH₂CH₂, Me), (B-265, H, HOOCCH₂CH₂CH₂CH₂, H), (B-266, H, HOOCCH₂CHCH₂CH₂, Cl), (B-267, H, HOOCCH₂CH₂CH₂CH₂, F), (B-268, H, HOOCCH₂CH₂CH₂CH₂, CF₃), (B-269, H, HOOCCH₂CH₂CH₂CH₂, Br), (B-270; H, HOOCCH₂CH₂CH₂CH₂, Me), (B-271, H, (Me)₂NCOCH₂CH₂CH₂CH₂, H), (B-272, H, (Me)₂NCOCH₂CH₂CH₂CH₂, Cl), (B-273, H, (Me)₂NCOCH₂CH₂CH₂CH₂, F), (B-274, H, (Me)₂NCOCH₂CH₂CH₂CH₂, CF₃), (B-275, H, (Me)₂NCOCH₂CH₂CH₂CH₂, Br), (B-276, H, (Me)₂NCOCH₂CH₂CH₂CH₂, Me), (B-277, H, (Me)₂NCOCH₂CH₂CH₂CH₂CH₂, H), (B-278, H, (Me)₂NCOCH₂CH₂CH₂CH₂CH₂, Cl), (B-279, H, (Me)₂NCOCH₂CH₂CH₂CH₂CH₂, F), (B-280, H, (Me)₂NCOCH₂CH₂CH₂CH₂CH₂, CF₃), (B.281, H, (Me)₂NCOCH₂CH₂CH₂CH₂CH₂, Br), (B-282, H, (Me)₂NCOCH₂CH₂CH₂CH₂CH₂, Me), (B-283, H, MeOCH₂, H), (B-284, H, MeOCH₂, Cl), (B-285, H, MeOCH₂, F), (B-286, H, MeOCH₂, CF₃), (B-287, H, MeOCH₂, Br), (B-288, H, MeOCH₂, Me), (B-289, H, EtOCH₂, H), (B-290, H, EtOCH₂, Cl), (B-291, H, EtOCH₂, F), (B-292, H, EtOCH₂, CF₃), (B-293, H, EtOCH₂, Br), (B-294, H, EtOCH₂, Me), (B-295, H, EtOCH₂CH₂, H), (B.296, H, EtOCH₂CH₂, Cl), (B-297, H, EtOCH₂CH₂, F), (B-298, H, EtOCH₂CH₂, CF₃), (B-299, H, EtOCH₂CH₂, Br), (B-300, H, EtOCH₂CH₂, Me), (B-301, H, MeOCH₂CH₂OCH₂CH₂, H), (B-302, H, MeOCH₂CH₂OCH₂CH₂, Cl), (B-303, H, MeOCH₂CH₂OCH₂CH₂, F), (B-304, H, MeOCH₂CH₂OCH₂CH₂, CF₃), (B-305, H, MeOCH₂CH₂OCH₂CH₂, Br), (B-306, H, MeOCH₂CH₂OCH₂CH₂, Me), (B-307, H, MeOCH₂CH₂, H), (B-308, H, MeOCH₂CH₂, Cl), (B-309, H, MeOCH₂CH₂, F), (B-310, H, MeOCH₂CH₂, CF₃), (B-311, H, MeOCH₂CH₂, Br), (B-312, H, MeOCH₂CH₂, Me), (B-313, H, HOCH₂, H), (B-314, H, HOCH₂, Cl), (B-315, H, HOCH₂, F), (B-316, H, HOCH₂, CF₃), (B-317, H, HOCH₂, Br), (B-318, H, HOCH₂, Me), (B-319, H, HOCH₂CH₂, H), (B-320, H, HOCH₂CH₂, Cl), (B-321, H, HOCH₂CH₂, F), (B-322, H, HOCH₂CH₂, CF₃), (B-323, H, HOCH₂CH₂, Br), (B-324, H, HOCH₂CH₂, Me), (B-325, H, HOCH₂CH₂CH₂, H), (B-326, H, HOCH₂CH₂CH₂, Cl), (B-327, H, HOCH₂CH₂CH₂, F), (B-328, H, HOCH₂CH₂CH₂, CF₃), (B-329, H, HOCH₂CH₂CH₂, Br), (B-330, H, HOCH₂CH₂CH₂, Me), (B-331, H, HOCH₂CH₂CH₂CH₂, H), (B-332, H, HOCH₂CH₂CH₂CH₂, Cl), (B-333, H, HOCH₂CH₂CH₂CH₂, F), (B-334, H, HOCH₂CH₂CH₂CH₂, CF₃), (B-335, H, HOCH₂CH₂CH₂CH₂, Br), (B-336, H, HOCH₂CH₂CH₂CH₂, Me), (B-337, H, HOCH₂CH₂CH₂CH₂CH₂, H), (B-338, H, HOCH₂CH₂CH₂CH₂CH₂, Cl), (B-339, H, HOCH₂CH₂CH₂CH₂CH₂, F), (B-340, H, HOCH₂CH₂CH₂CH₂CH₂, CF₃), (B-341, H, HOCH₂CH₂CH₂CH₂CH₂, Br), (B-342, H, HOCH₂CH₂CH₂CH₂CH₂, Me), (B-343, H, HOCH₂CH₂OCH₂CH₂, H), (B-344, H, HOCH₂CH₂OCH₂CH₂, Cl), (B-345, H. HOCH₂CH₂OCH₂CH₂, F), (B-346, H, HOCH₂CH₂OCH₂CH₂, CF₃), (B-347, H, HOCH₂CH₂OCH₂CH₂, Br), (B-348, H, HOCH₂CH₂OCH₂CH₂, Me), (B-349, H, (Me)₂N, H), (B-350, H, (Me)₂N, Cl), (B-351, H, (Me)₂N, F), (B-352, H, (Me)₂N, CF₃), (B-353, H, (Me)₂N, Br), (B-354, H, (Me)₂N, Me), (B-355, H, piperidin-4-yl-methyl, H), (B-356, H, piperidin-4-yl-methyl, Cl), (B-357, H, piperidin-4-yl-methyl, F), (B-358, H, piperidin-4-yl-methyl, CF₃), (B-359, H, piperidin-4-yl-methyl, Br), (B-360, H, piperidin-4-yl-methyl, Me), (B-361, H, cyclohexylmethyl, H), (B-362, H, cyclohexylmethyl, Cl), (B-363, H, cyclohexylmethyl, F), (B-364, H, cyclohexylmethyl, CF₃), (B-365, H, cyclohexylmethyl, Br), (B-366, H, cyclohexylmethyl, Me), (B-367, F, H, H), (B-368, F, H, Cl), (B-369, F, H, F), (B-370, F, H, CF₃), (B-371, F, H, Br), (B-372, F, H, Me), (B-373, F, F, H), (B-374, F, F, Cl), (B-375, F, F, F), (B-376, F, F, CF₃), (B-377, F, F, Br), (B-378, F, F, Me), (B-379, F, Cl, H), (B-380, F, Cl, Cl), (B-381, F, Cl, F), (B-382, F, Cl, CF₃), (B-383, F, Cl, Br), (B-384, F, Cl, Me), (B-385, F, Me, H), (B-386, F, Me, Cl), (B-387, F, Me, F), (B-388, F, Me, CF₃), (B-389, F, Me, Br), (B-390, F, Me, Me), (B-391, F, Et, H), (B-392, F, Et, Cl), (B-393, F, Et, F), (B-394, F, Et, CF₃), (B-395, F, Et, Br), (B-396, F, Et, Me), (B-397, F, n-Pr, H), (B-398, F, n-Pr, Cl), (B-399, F, n-Pr, F), (B-400, F, n-Pr, CF₃), (B-401, F, n-Pr, Br), (B-402, F, n-Pr, Me), (B-403, F, c-Pr, H), (B-404, F, c-Pr, Cl), (B-405, F, c-Pr, F), (B-406, F, c-Pr, CF₃)_{,} (B-407, F, c-Pr, Br), (B-408, F, c-Pr, Me), (B-409, F, i-Pr, H), (B-410, F, i-Pr, Cl), (B-411, F, i-Pr, F), (B-412, F, i-Pr, CF₃), (B-413, F, i-Pr, Br), (B-414, F, i-Pr, Me), (B-415, F, n-Bu, H), (B-416, F, n-Bu, Cl), (B-417, F, n-Bu, F), (B-418, F, n-Bu, CF₃), (B-419, F, n-Bu, Br), (B-420, F, n-Bu, Me), (B-421, F, i-Bu, H), (B-422, F, i-Bu, Cl), (B-423, F, i-Bu, F), (B-424, F, i-Bu, CF₃), (B-425, F, i-Bu, Br), (B-426, F, i-Bu, Me), (B-427, F, sec-Bu, H), (B-428, F, sec-Bu, Cl), (B-429, F, sec-Bu, F), (B-430, F, sec-Bu, CF₃), (B-431, F, sec-Bu, Br), (B-432, F, sec-Bu, Me), (B-433, F, n-Pen, H), (B-434, F, n-Pen, Cl), (B-435, F, n-Pen, F), (B-436, F, n-Pen, CF₃), (B-437, F, n-Pen, Br), (B-438, F, n-Pen, Me), (B-439, F, c-Pen, H), (B-440, F, c-Pen, Cl), (B-441, F, c-Pen, F), (B-442, F, c-Pen, CF₃), (B-443, F, c-Pen, Br), (B-444, F, c-Pen, Me), (B-445, F, n-Hex, H), (B-446, F, n-Hex, Cl), (B-447, F, n-Hex, F), (B-448, F, n-Hex, CF₃)_{,} (B-449, F, n-Hex, Br), (B-450, F, n-Hex, Me), (B-451, F, c-Hex, H), (B-452, F, c-Hex, Cl), (B-453, F, c-Hex, F), (B-454, F, c-Hex, CF₃), (B-455, F, c-Hex, Br), (B-456, F, c-Hex, Me), (B-457, F, OH, H), (B-458, F, OH, Cl), (B-459, F, OH, F), (B-460, F, OH, CF₃), (B-461, F, OH, Br), (B-462, F, OH, Me), (B-463, F, MeO, H), (B-464, F, MeO, Cl), (B-465, F, MeO, F), (B-466, F, MeO, CF₃), (B-467, F, MeO, Br), (B-468, F, MeO, Me), (B-469, F, EtO, H), (B-470, F, EtO, Cl), (B-471, F, EtO, F), (B-472, F, EtO, CF₃), (B-473, F, EtO, Br), (B-474, F, EtO, Me), (B-475, F, n-PrO, H), (B-476, F, n-PrO, Cl), (B-477, F, n-PrO, F), (B-478, F, n-PrO, CF₃), (B-479, F, n-PrO, Br), (B-480, F, n-PrO, Me), (B-481, F, PhO, H), (B-482, F, PhO, Cl), (B-483, F, PhO, F), (B-484, F, PhO, CF₃), (B-485, F, PhO, Br), (B-486, F, PhO, Me), (8-487, F, BnO, H), (B-488, F, BnO, Cl), (B-489, F, BnO, F), (B-490, F, BnO, CF₃), (B-491, F, BnO, Br), (B-492, F, BnO, Me), (B-493, F, PhCH₂CH₂O, H), (B-494, F, PhCH₂CH₂O, Cl), (B-495, F, PhCH₂CH₂O, F), (B-496, F, PhCH₂CH₂O, CF₃), (B-497, F, PhCH₂CH₂O, Br), (B-498, F, PhCH₂CH₂O, Me), (B-499, F, CF₃, H), (B-500, F, CF₃, Cl), (B-501, F, CF₃, F), (B-502, F, CF₃, CF₃), (B-503, F, CF₃, Br), (B-504, F, CF₃, Me), (B-505, F, CF₃O, H), (B-506, F, CF₃O, Cl), (B-507, F, CF₃O, F), (B-508, F, CF₃O, CF₃), (B-509, F, CF₃O, Br), (B-510, F, CF₃O, Me), (B-511, F, Ph, H), (B-512, F, Ph, Cl), (B-513, F, Ph, F), (B-514, F, Ph, CF₃), (B-515, F, Ph, Br), (B-516, F, Ph, Me), (B-517, F, 4-F-Ph, H), (B-518, F, 4-F-Ph, Cl), (B-519, F, 4-F-Ph, F), (B-520, F, 4-F-Ph, CF₃), (B-521, F, 4-F-Ph, Br), (B-522, F, 4-F-Ph, Me), (B-523, F, 4-CF₃-Ph, H), (B-524, F, 4-CF₃-Ph, Cl), (B-525, F, 4-CF₃-Ph, F), (B-526, F, 4-CF₃-Ph, CF₃), (B-527, F, 4-CF₃-Ph, Br), (B-528, F, 4-CF₃-Ph, Me), (B-529, F, 4-(Me)₂N-Ph, H), (B-530, F, 4-(Me)₂N-Ph, Cl), (B-531, F, 4-(Me)₂N-Ph, F), (B-532, F, 4-(Me)₂N-Ph, CF₃), (B-533, F, 4-(Me)₂N-Ph, Br), (B-534, F, 4-(Me)₂N-Ph, Me), (B-535, F, 4-OH-Ph, H), (B-536, F, 4-OH-Ph, Cl), (B-537, F, 4-OH-Ph, F), (B-538, F, 4-OH-Ph, CF₃), (B-539, F, 4-OH-Ph, Br), (B-540, F, 4-OH-Ph, Me), (B-541, F, 3,4-di-F-Ph, H), (B-542, F, 3,4-di-F-Ph, Cl), (B-543, F, 3,4-di-F-Ph, F), (B-544, F, 3,4-di-F-Ph, CF₃), (B-545, F, 3,4-di-F-Ph, Br), (B-546, F, 3,4-di-F-Ph, Me), (B-547, F, 4-COOH-Ph, H), (B-548, F, 4-COOH-Ph, Cl), (B-549, F, 4-COOH-Ph, F), (B-550, F, 4-COOH-Ph, CF₃), (B-551, F, 4-COOH-Ph, Br), (B-552, F, 4-COOH-Ph, Me), (B-553, F, Bn, H), (B-554, F, Bn, Cl), (B-555, F, Bn, F), (B-556, F, Bn, CF₃), (B-557, F, Bn, Br), (B-558, F, Bn, Me), (B-559, F, 4-F-Bn, H), (B-560, F, 4-F-Bn, Cl), (B-561, F, 4-F-Bn, F), (B-562, F, 4-F-Bn, CF₃), (B-563, F, 4-F-Bn, Br), (B-564, F, 4-F-Bn, Me), (B-565, F, 2-Py, H), (B.566, F, 2-Py, Cl), (B-567, F, 2-Py, F), (B-568, F, 2-Py, CF₃), (B-569, F, 2-Py, Br), (B-570, F, 2-Py, Me), (B-571, F, 3-Py, H), (B-572, F, 3-Py, Cl), (B-573, F, 3-Py, F), (B-574, F, 3-Py, CF₃), (B-575, F, 3-Py, Br), (B-576, F, 3-Py, Me), (B-577, F, 4-Py, H), (B-578, F, 4-Py, Cl), (B-579, F, 4-Py, F), (B-580, F, 4-Py, CF₃), (B-581, F, 4-Py, Br), (B-582, F, 4-Py, Me), (B-583, F, 2-Th, H), (B-584, F, 2-Th, Cl), (B-585, F, 2-Th, F), (B-586, F, 2-Th, CF₃)_{,} (B-587, F, 2-Th, Br), (B-588, F, 2-Th, Me), (B-589, F, 3-Th, H), (B-590, F, 3-Th, Cl), (B-591, F, 3-Th, F), (8-592, F, 3-Th, CF₃), (B-593, F, 3-Th, Br), (B-594, F, 3-Th, Me), (B-595, F, Pyrazol-2-yl, H), (B-596, F, Pyrazol-2-yl, Cl), (B-597, F, Pyrazol-2-yl, F), (B-598, F, Pyrazol-2-yl, CF₃), (B-599, F, Pyrazol-2-yl, Br), (B-600, F, Pyrazol-2-yl, Me), (B-601, F, Pyrazol-3-yl, H), (B-602, F, Pyrazol-3-yl, Cl), (B-603, F, Pyrazol-3-yl, F), (B-604, F, Pyrazol-3-yl, CF₃), (B-605, F, Pyrazol-3-yl, Br), (B-606, F, Pyrazol-3-yl, Me), (B-607, F, pyrimidin-2-yl, H), (B-608, F, pyrimidin-2-yl, Cl), (B-609, F, pyrimidin-2-yl, F), (B-610, F, pyrimidin-2-yl, CF₃), (B-611, F, pyrimidin-2-yl, Br), (B-612, F, pyrimidin-2-yl, Me), (B-613, F, pyrimidin-4-yl, H), (B-614, F, pyrimidin-4-yl, Cl), (B-615, F, pyrimidin-4-yl, F), (B-616, F, pyrimidin-4-yl, CF₃), (B-617, F, pyrimidin-4-yl, Br), (B-618, F, pyrimidin-4-yl, Me), (B-619, F, pyrimidin-5-yl, H), (B-620, F, pyrimidin-5-yl, Cl), (B-621, F, pyrimidin-5-yl, F), (B-622, F, pyrimidin-5-yl, CF₃), (B-623, F, pyrimidin-5-yl, Br), (B-624, F, pyrimidin-5-yl, Me), (B-625, F, HOOCCH₂CH₂CH₂, H), (B-626, F, HOOCCH₂CH₂CH₂, Cl), (B-627, F, HOOCCH₂CH₂CH₂, F), (B-628, F, HOOCCH₂CH₂CH₂, CF₃), (B-629, F, HOOCCH₂CH₂CH₂, Br), (B-630, F, HOOCCH₂CH₂CH₂, Me), (B-631, F, HOOCCH₂CH₂CH₂CH₂, H), (B-632, F, HOOCCH₂CH₂CH₂CH₂, Cl), (B-633, F, HOOCCH₂CH₂CH₂CH₂, F), (B-634, F, HOOCCH₂CH₂CH₂CH₂, CF₃), (B-635, F, HOOCCH₂CH₂CH₂CH₂, Br), (B-636, F, HOOCCH₂CH₂CH₂CH₂, Me), (B-637, F, (Me)₂NCOCH₂CH₂CH₂CH₂, H), (B-638, F, (Me)₂NCOCH₂CH₂CH₂CH₂, Cl), (B-639, F, (Me)₂NCOCH₂CH₂CH₂CH₂, F), (B-640, F, (Me)₂NCOCH₂CH₂CH₂CH₂, CF₃), (B-641, F, (Me)₂NCOCH₂CH₂CH₂CH₂, Br), (B-642, F, (Me)₂NCOCH₂CH₂CH₂CH₂, Me), (B-643, F, (Me)₂NCOCH₂CH₂CH₂CH₂CH₂, H), (B-644, F, (Me)₂NCOCH₂CH₂CH₂CH₂CH₂, Cl), (B-645, F, (Me)₂NCOCH₂,CH₂CH₂CH₂CH₂, F), (B-646, F, (Me)₂NCOCH₂CH₂CH₂CH₂CH₂, CF₃), (B-647, F, (Me)₂NCOCH₂CH₂CH₂CH₂CH₂, Br), (B-648, F, (Me)₂NCOCH₂CH₂CH₂CH₂CH₂, Me), (B-649, F, MeOCH₂, H), (B-650, F, MeOCH₂, Cl), (B-651, F, MeOCH₂, F), (B-652, F, MeOCH₂, CF₃), (B-653, F, MeOCH₂, Br), (B-654, F, MeOCH₂, Me), (B-655, F, EtOCH₂, H), (B-656, F, EtOCH₂, Cl), (B-657, F, EtOCH₂, F), (B-658, F, EtOCH₂, CF₃), (B-659, F, EtOCH₂, Br), (B-660, F, EtOCH₂, Me), (B-661, F, EtOCH₂CH₂, H), (B-662, F, EtOCH₂CH₂, Cl), (B-663, F, EtOCH₂CH₂, F), (B-664, F, EtOCH₂CH₂, CF₃), (B-665, F, EtOCH₂CH₂, Br), (B-666, F, EtOCH₂CH₂, Me), (B-667, F, MeOCH₂CH₂OCH₂CH₂, H), (B-668, F, MeOCH₂CH₂OCH₂CH₂, Cl), (B-669, F, MeOCH₂CH₂OCH₂CH₂, F), (B-670, F, MeOCH₂CH₂OCH₂CH₂, CF₃), (B-671, F, MeOCH₂CH₂OCH₂CH₂, Br), (B-672, F, MeOCH₂CH₂OCH₂CH₂, Me), (B-673, F, MeOCH₂CH₂, H), (B-674, F, MeOCH₂CH₂, Cl), (B-675, F, MeOCH₂CH₂, F), (B-676, F, MeOCH₂CH₂, CF₃), (B-677, F, MeOCH₂CH₂, Br), (B-678, F, MeOCH₂CH₂, Me), (B-679, F, HOCH_{2,} H), (B-680, F, HOCH₂, Cl), (B-681, F, HOCH₂, F), (B-682, F, HOCH₂, CF₃), (B-683, F, HOCH₂, Br), (B-684, F, HOCH₂, Me), (B-685, F, HOCH₂CH₂, H), (B-686, F, HOCH₂CH₂, Cl), (B-687, F, HOCH₂CH₂, F), (B-688, F, HOCH₂CH₂, CF₃), (B-689, F, HOCH₂CH₂, Br), (B-690, F, HOCH₂CH₂, Me), (B-691, F, HOCH₂CH₂CH₂, H), (B-692, F, HOCH₂CH₂CH₂, Cl), (B-693, F, HOCH₂CH₂CH₂, F), (B-694, F, HOCH₂CH₂CH₂, CF₃), (B-695, F, HOCH₂CH₂CH₂, Br), (B-696, F, HOCH₂CH₂CH₂, Me), (B-697, F, HOCH₂CH₂CH₂CH₂, H), (B-698, F, HOCH₂CH₂CH₂CH₂, Cl), (B-699, F, HOCH₂CH₂CH₂CH₂, F), (B-700, F, HOCH₂CH₂CH₂CH₂, CF₃), (B-701, F, HOCH₂CH₂CH₂CH₂, Br), (B-702, F, HOCH₂CH₂CH₂CH₂, Me), (B-703, F, HOCH₂CH₂CH₂CH₂CH₂, H), (B-704, F, HOCH₂CH₂CH₂CH₂CH₂, Cl), (B-705, F, HOCH₂CH₂CH₂CH₂CH₂, F), (B-706, F, HOCH₂CH₂CH₂CH₂CH₂, CF₃), (B-707, F, HOCH₂CH₂CH₂CH₂, Br), (B-708, F, HOCH₂CH₂CH₂CH₂CH₂, Me)_{,} (B-709, F, HOCH₂CH₂OCH₂CH₂, H), (B-710, F, HOCH₂CH₂OCH₂CH₂, Cl), (B-711, F, HOCH₂CH₂OCH₂CH₂, F), (B-712, F, HOCH₂CH₂OCH₂CH₂, CF₃), (B-713, F, HOCH₂CH₂OCH₂CH₂, Br), (B-714, F, HOCH₂CH₂OCH₂CH₂, Me), (B-715, F, (Me)₂N, H), (B-716, F, (Me)₂N, Cl), (B-717, F, (Me)₂N, F), (B-718, F, (Me)₂N, CF₃), (B-719, F, (Me)₂N, Br), (B-720, F, (Me)₂N, Me), (B-721, F, piperidin-4-yl-methyl, H), (B-722, F, piperidin-4-yl-methyl, Cl), (B-723, F, piperidin-4-yl-methyl, F), (B-724, F, piperidin-4-yl-methyl, CF₃), (B-725, F, piperidin-4-yl-methyl, Br), (B-726, F, piperidin-4-yl-methyl, Me), (B-727, F, cyclohexylmethyl, H), (B-728, F, cyclohexylmethyl, Cl), (B-729, F, cyclohexylmethyl, F), (B-730, F, cyclohexylmethyl, CF₃), (B-731, F, cyclohexylmethyl, Br), (B-732, F, cyclohexylmethyl, Me), (B-733, Me, H, H), (B-734, Me, H, Cl), (B-735, Me, H, F), (B-736, Me, H, CF₃), (B-737, Me, H, Br), (B-738, Me, H, Me), (B-739, Me, F, H), (B-740, Me, F, Cl), (B-741, Me, F, F), (B-742, Me, F, CF₃), (B-743, Me, F, Br), (B-744, Me, F, Me), (B-745, Me, Cl, H), (B-746, Me, Cl, Cl), (B-747, Me, Cl, F), (B-748, Me, Cl, CF₃), (B-749, Me, Cl, Br), (B-750, Me, Cl, Me), (B-751, Me, Me, H), (B-752, Me, Me, Cl), (B-753, Me, Me, F), (B-754, Me, Me, CF₃), (B-755, Me, Me, Br), (B-756, Me, Me, Me), (B-757, Me, Et, H), (B-758, Me, Et, Cl), (B-759, Me, Et, F), (B-760, Me, Et, CF₃), (B-761, Me, Et, Br), (B-762, Me, Et, Me), (B-763, Me, n-Pr, H), (B-764, Me, n-Pr, Cl), (B-765, Me, n-Pr, F), (B-766, Me, n-Pr, CF₃), (B-767, Me, n-Pr, Br), (B-768, Me, n-Pr, Me), (B-769, Me, c-Pr, H), (B-770, Me, c-Pr, Cl), (B-771, Me, c-Pr, F), (B-772, Me, c-Pr, CF₃), (B-773, Me, c-Pr, Br), (B-774, Me, c-Pr, Me), (B-775, Me, i-Pr, H), (B-776, Me, i-Pr, Cl), (B-777, Me, i-Pr, F), (B-778, Me, i-Pr, CF₃), (B-779, Me, i-Pr, Br), (B-780, Me, i-Pr, Me), (B-781, Me, n-Bu, H), (B-782, Me, n-Bu, Cl), (B-783, Me, n-Bu, F), (B-784, Me, n-Bu, CF₃), (B-785, Me, n-Bu, Br), (B-786, Me, n-Bu, Me), (B-787, Me, i-Bu, H), (B-788, Me, i-Bu, Cl), (B-789, Me, i-Bu, F), (B-790, Me, i-Bu, CF₃), (B-791, Me, i-Bu, Br), (B-792, Me, i-Bu, Me), (B-793, Me, sec-Bu, H), (B-794, Me, sec-Bu, Cl), (B-795, Me, sec-Bu, F), (B-796, Me, sec-Bu, CF₃), (B-797, Me, sec-Bu, Br), (B-798, Me, sec-Bu, Me), (B-799, Me, n-Pen, H), (B-800, Me, n-Pen, Cl), (B-801, Me, n-Pen, F), (B-802, Me, n-Pen, CF₃), (B-803, Me, n-Pen, Br), (B-804, Me, n-Pen, Me), (B-805, Me, c-Pen, H), (B-806, Me, c-Pen, Cl), (B-807, Me, c-Pen, F), (B-808, Me, c-Pen, CF₃), (B-809, Me, c-Pen, Br), (B-810, Me, c-Pen, Me), (B-811, Me, n-Hex, H), (B-812, Me, n-Hex, Cl), (B-813, Me, n-Hex, F), (B-814, Me, n-Hex, CF₃), (B-815, Me, n-Hex, Br), (B-816, Me, n-Hex, Me), (B-817, Me, c-Hex, H), (B-818, Me, c-Hex, Cl), (B-819, Me, c-Hex, F), (B-820, Me, c-Hex, CF₃), (B-821, Me, c-Hex, Br), (B-822, Me, c-Hex, Me), (B-823, Me, OH, H), (B-824, Me, OH, Cl), (B-825, Me, OH, F), (B-826, Me, OH, CF₃), (B-827, Me, OH, Br), (B-828, Me, OH, Me), (B-829, Me, MeO, H), (B-830, Me, MeO, Cl), (B-831, Me, MeO, F), (B-832, Me, MeO, CF₃), (B-833, Me, MeO, Br), (B-834, Me, MeO, Me), (B-835, Me, EtO, H), (B-836, Me, EtO, Cl), (B-837, Me, EtO, F), (B-838, Me, EtO, CF₃), (B-839, Me, EtO, Br), (B-840, Me, EtO, Me), (B-841, Me, n-PrO, H), (B-842, Me, n-PrO, Cl), (B-843, Me, n-PrO, F), (B-844, Me, n-PrO, CF₃), (B-845. Me, n-PrO, Br), (B-846, Me, n-PrO, Me), (B-847, Me, PhO, H), (B-848, Me, PhO, Cl), (B-849, Me, PhO, F), (B-850, Me, PhO, CF₃), (B-851, Me, PhO, Br), (B-852, Me, PhO, Me), (B-853, Me, BnO, H), (B-854, Me, BnO, Cl), (B-855, Me, BnO, F), (B-856, Me, BnO, CF₃), (B-857, Me, BnO, Br), (B-858, Me, BnO, Me), (B-859, Me, PhCH₂CH₂O, H), (B-860, Me, PhCH₂CH₂O, Cl), (B-861, Me, PhCH₂CH₂O, F), (B-862, Me, PhCH₂CH₂O, CF₃), (B-863, Me, PhCH₂CH₂O, Br), (B-864, Me, PhCH₂CH₂O, Me), (B-865, Me, CF₃, H), (B-866, Me, CF₃, Cl), (B-867, Me, CF₃, F), (B-868, Me, CF₃, CF₃), (B-869, Me, CF₃, Br), (B-870, Me, CF₃, Me), (B-871, Me, CF₃O, H), (B-872, Me, CF₃O, Cl), (B-873, Me, CF₃O, F), (B-874, Me, CF₃O, CF₃), (B-875, Me, CF₃O, Br), (B-876, Me, CF₃O, Me), (B-877, Me, Ph, H), (B-878, Me, Ph, Cl), (B-879, Me, Ph, F), (B-880, Me, Ph, CF₃), (B-881, Me, Ph, Br), (B-882, Me, Ph, Me), (B-883, Me, 4-F-Ph, H), (B-884, Me, 4-F-Ph, Cl), (B-885, Me, 4-F-Ph, F), (B-886, Me, 4-F-Ph, CF₃), (B-887, Me, 4-F-Ph, Br), (B-888, Me, 4-F-Ph, Me), (B-889, Me, 4-CF₃-Ph, H), (B-890, Me, 4-CF₃-Ph, Cl), (B-891, Me, 4-CF₃-Ph, F), (B-892, Me, 4-CF₃-Ph, CF₃), (B-893, Me, 4-CF₃-Ph, Br), (B-894, Me, 4-CF₃-Ph, Me), (B-895, Me, 4-(Me)₂N-Ph, H), (B-896, Me, 4-(Me)₂N-Ph, Cl), (B-897, Me, 4-(Me)₂N-Ph, F), (B-898, Me, 4-(Me)₂N-Ph, CF₃), (B-899, Me, 4-(Me)₂N-Ph, Br), (B-900, Me, 4-(Me)₂N-Ph, Me), (B-901, Me, 4-OH-Ph, H), (B-902, Me, 4-OH-Ph, Cl), (B-903, Me, 4-OH-Ph, F), (B-904, Me, 4-OH-Ph, CF₃), (B-905, Me, 4-OH-Ph, Br), (B-906, Me, 4-OH-Ph, Me), (B-907, Me, 3,4-di-F-Ph, H), (B-908, Me, 3,4-di-F-Ph, Cl), (B-909, Me, 3,4-di-F-Ph, F), (B-910, Me, 3,4-di-F-Ph, CF₃), (B-911, Me, 3,4-di-F-Ph, Br), (B-912, Me, 3,4-di-F-Ph, Me), (B-913, Me, 4-COOH-Ph, H), (B-914, Me, 4-COOH-Ph, Cl), (B-915, Me, 4-COOH-Ph, F), (B-916, Me, 4-COOH-Ph, CF₃), (B-917, Me, 4-COOH-Ph, Br), (B-918, Me, 4-COOH-Ph, Me), (B-919, Me, Bn, H), (B-920, Me, Bn, Cl), (B-921, Me, Bn, F), (B-922, Me, Bn, CF₃), (B-923, Me, Bn, Br), (B-924, Me, Bn, Me), (B-925, Me, 4-F-Bn, H), (B-926, Me, 4-F-Bn, Cl), (B-927, Me, 4-F-Bn, F), (B-928, Me, 4-F-Bn, CF₃), (B-929, Me, 4-F·Bn, Br), (B-930, Me, 4-F-Bn, Me), (B-931, Me, 2-Py, H), (B-932, Me, 2-Py, Cl), (B-933, Me, 2-Py, F), (B-934, Me, 2-Py, CF₃), (B-935, Me, 2-Py, Br), (B-936, Me, 2-Py, Me), (B-937, Me, 3-Py, H), (B-938, Me, 3-Py, Cl), (B-939, Me, 3-Py, F), (B-940, Me, 3-Py, CF₃), (B-941, Me, 3-Py, Br), (B-942, Me, 3-Py, Me), (B-943, Me, 4-Py, H), (B-944, Me, 4-Py, Cl), (B-945, Me, 4-Py, F), (B-946, Me, 4-Py, CF₃), (B-947, Me, 4-Py, Br), (B-948, Me, 4-Py, Me), (B-949, Me, 2-Th, H), (B-950, Me, 2-Th, Cl), (B-951, Me, 2-Th, F), (B-952, Me, 2-Th, CF₃), (B-953, Me, 2-Th, Br), (B-954, Me, 2-Th, Me), (B-955, Me, 3-Th, H), (B-956, Me, 3-Th, Cl), (B-957, Me, 3-Th, F), (B-958, Me, 3-Th, CF₃), (B-959, Me, 3-Th, Br), (B-960, Me, 3-Th, Me), (B-961, Me, Pyrazol-2-yl, H), (B-962, Me, Pyrazol-2-yl, Cl), (B-963, Me, Pyrazol-2-yl, F), (B-964, Me, Pyrazol-2-yl, CF₃), (B-965, Me, Pyrazol-2-yl, Br), (B-966, Me, Pyrazol-2-yl, Me), (B-967, Me, Pyrazol-3-yl, H), (B-968, Me, Pyrazol-3-yl, Cl), (B-969, Me, Pyrazol-3-yl, F), (B-970, Me, Pyrazol-3-yl, CF₃), (B-971, Me, Pyrazol-3-yl, Br), (B-972, Me, Pyrazol-3-yl, Me), (B-973, Me, pyrimidin-2-yl, H), (B-974, Me, pyrimidin-2-yl, Cl), (B-975, Me, pyrimidin-2-yl, F), (B-976, Me, pyrimidin-2-yl, CF₃), (B-977, Me, pyrimidin-2-yl, Br), (B-978, Me, pyrimidin-2-yl, Me), (B-979, Me, pyrimidin-4-yl, H), (B-980, Me, pyrimidin-4-yl, Cl), (B-981, Me, pyrimidin-4-yl, F), (B-982, Me, pyrimidin-4-yl, CF₃), (B-983, Me, pyrimidin-4-yl, Br), (B-984, Me, pyrimidin-4-yl, Me), (B-985, Me, pyrimidin-5-yl, H), (B-986, Me, pyrimidin-5-yl, Cl), (B-987, Me, pyrimidin-5-yl, F), (B-988, Me, pyrimidin-5-yl, CF₃), (B-989, Me, pyrimidin-5-yl, Br), (B-990, Me, pyrimidin-5-yl, Me), (B-991, Me, HOOCCH₂CH₂CH₂, H), (B-992, Me, HOOCCH₂CH₂CH₂, Cl), (B-993, Me, HOOCCH₂CH₂CH₂, F), (B-994, Me, HOOCCH₂CH₂CH₂, CF₃), (B-995, Me, HOOCCH₂CH₂CH₂, Br), (B-996, Me, HOOCCH₂CH₂CH₂, Me), (B-997, Me, HOOCCH₂CH₂CH₂CH₂, H), (B-998, Me, HOOCCH₂CH₂CH₂CH₂, Cl), (B-999, Me, HOOCCH₂CH₂CH₂CH₂, F), (B-1000, Me, HOOCCH₂CH₂CH₂CH₂, CF₃), (B-1001, Me, HOOCCH₂CH₂CH₂CH₂, Br), (B-1002, Me, HOOCCH₂CH₂CH₂CH₂, Me), (B-1003, Me, (Me)₂NCOCH₂CH₂CH₂CH₂, H), (B-1004, Me, (Me)₂NCOCH₂CH₂CH₂CH₂, Cl), (B-1005, Me, (Me)₂NCOCH₂CH₂CH₂CH₂, F), (B-1006, Me, (Me)₂NCOCH₂CH₂CH₂CH₂, CF₃), (B-1007, Me, (Me)₂NCOCH₂CH₂CH₂CH₂, Br), (B-1008, Me, (Me)₂NCOCH₂CH₂CH₂CH₂, Me), (B-1009, Me, (Me)₂NCOCH₂CH₂CH₂CH₂CH₂, H), (B-1010, Me, (Me)₂NCOCH₂CH₂CH₂CH₂CH₂, Cl), (B-1011, Me, (Me)₂NCOCH₂CH₂CH₂CH₂CH₂, F), (B-1012, Me, (Me)₂NCOCH₂CH₂CH₂CH₂CH₂, CF₃), (B-1013, Me, (Me)₂NCOCH₂CH₂CH₂CH₂CH₂, Br), (B-1014, Me, (Me)₂NCOCH₂CH₂CH₂CH₂CH₂, Me), (B-1015, Me, MeOCH₂, H), (B-1016, Me, MeOCH₂, Cl)_{,} (B-1017, Me, MeOCH₂, F), (B-1018, Me, MeOCH₂, CF₃), (B-1019, Me, MeOCH₂, Br), (B-1020, Me, MeOCH₂, Me), (B-1021, Me, EtOCH₂, H), (B-1022, Me, EtOCH₂, Cl), (B-1023, Me, EtOCH₂, F), (B-1024, Me, EtOCH₂, CF₃), (B-1025, Me, EtOCH₂, Br), (B-1026, Me, EtOCH2, Me), (B-1027, Me, EtOCH₂CH₂, H), (B-1028, Me, EtOCH₂CH₂, Cl), (B-1029, Me, EtOCH₂CH₂, F), (B-1030, Me, EtOCH₂CH₂, CF₃), (B-1031, Me, EtOCH₂CH₂, Br), (B-1032, Me, EtOCH₂CH₂, Me), (B-1033, Me, MeOCH₂CH2OCH₂CH₂, H), (B-1034, Me, MeOCH₂CH₂OCH₂CH₂, Cl), (B-1035, Me, MeOCH₂CH₂OCH₂CH₂, F), (B-1036, Me, MeOCH₂CH₂OCH₂CH₂, CF₃), (B-1037, Me, MeOCH₂CH₂OCH₂CH₂, Br), (B-1038, Me, MeOCH₂CH₂OCH₂CH₂, Me), (B-1039, Me, MeOCH₂CH₂, H), (B-1040, Me, MeOCH₂CH₂, Cl), (B-1041, Me, MeOCH₂CH₂, F), (B-1042, Me, MeOCH₂CH₂, CF₃), (B-1043, Me, MeOCH₂CH₂, Br), (B-1044, Me, MeOCH₂CH₂, Me), (B-1045, Me, HOCH₂, H), (B-1046, Me, HOCH₂, Cl), (B-1047, Me, HOCH₂, F), (B-1048, Me, HOCH₂, CF₃), (B-1049, Me, HOCH₂, Br), (B-1050, Me, HOCH₂, Me), (B-1051, Me, HOCH₂CH₂, H), (B-1052, Me, HOCH₂CH₂, Cl), (B-1053, Me, HOCH₂CH₂, F), (B-1054, Me, HOCH₂CH₂, CF₃), (B-1055, Me, HOCH₂CH₂, Br), (B-1056, Me, HOCH₂CH₂, Me), (B-1057, Me, HOCH₂CH₂CH₂, H), (B-1058, Me, HOCH₂CH₂CH₂, Cl), (B-1059, Me, HOCH₂CH₂CH₂, F), (B-1060, Me, HOCH₂CH₂CH₂, CF₃), (B-1061, Me, HOCH₂CH₂CH₂; Br), (B-1062, Me, HOCH₂CH₂CH₂, Me), (B-1063, Me, HOCH₂CH₂CH₂CH₂, H), (B-1064, Me, HOCH₂CH₂CH₂CH₂, Cl), (B-1065, Me, HOCH₂CH₂CH₂CH₂, F), (B-1066, Me, HOCH₂CH₂CH₂CH₂, CF₃), (B-1067, Me, HOCH₂CH₂CH₂CH₂, Br), (B-1068, Me, HOCH₂CH₂CH₂CH₂, Me), (B-1069, Me, HOCH₂CH₂CH₂CH₂CH₂, H), (B-1070, Me, HOCH₂CH₂CH₂CH₂CH₂, Cl), (B-1071, Me, HOCH₂CH₂CH₂CH₂CH₂, F), (B-1072, Me, HOCH₂CH₂CH₂CH₂CH₂, CF₃), (B-1073, Me, HOCH₂CH₂CH₂CH₂CH₂, Br), (B-1074, Me, HOCH₂CH₂CH₂CH₂CH₂, Me), (B-1075, Me, HOCH₂CH₂OCH₂CH₂, H), (B-1076, Me, HOCH₂CH₂OCH₂CH₂, Cl), (B-1077, Me, HOCH₂CH₂OCH₂CH₂, F), (B-1078, Me, HOCH₂CH₂OCH₂CH₂, CF₃), (B-1079, Me, HOCH₂CH₂OCH₂CH₂, Br), (B-1080, Me, HOCH₂CH₂OCH₂CH₂, Me), (B-1081, Me, (Me)₂N, H), (B-1082, Me, (Me)₂N, Cl), (B-1083, Me, (Me)₂N, F), (B-1084, Me, (Me)₂N, CF₃), (B-1085, Me, (Me)₂N, Br), (B-1086, Me, (Me)₂N, Me), (B-1087, Me, piperidin-4-yl-methyl, H), (B-1088, Me, piperidin-4-yl-methyl, Cl), (B-1089, Me, piperidin-4-yl-methyl, F), (B-1090, Me, piperidin-4-yl-methyl, CF₃), (B-1091, Me, piperidin-4-yl-methyl, Br), (B-1092, Me, piperidin-4-yl-methyl, Me), (B-1093, Me, cyclohexylmethyl, H), (B-1094, Me, cyclohexylmethyl, Cl), (B-1095, Me, cyclohexylmethyl, F), (B-1096, Me, cyclohexylmethyl, CF₃), (B-1097, Me, cyclohexylmethyl, Br), (B-1098, Me, cyclohexylmethyl, Me)

### Test Examples

### Test Example 1 Isolation and purification of Thrombopoietin (TPO)

Human TPO and mouse TPO were purchased from R&D Systems.

### Test Example 2 The thrombopoietic activity

The TPO dependent BaF/hTPOR cell line which was established by introducing human TPO receptor into BaF-B03 cells according to Collins et al (J. Cell. Physiol., 137:293-298 (1988)) was used to test the thrombopoietic activity of the present compound. The DNA sequences and encoded peptide sequences for human TPO receptor have been described by Vigon et al (Proc. Natl. Acad. Sci. USA, 89:5640-5644 (1992)). TPO dose not have any ability to support proliferation of interlukin-3 dependent parental cell line BaF-B03. BAF/hTPOR cells were maintained in RPMI medium and 10% WEHI-3 conditioned medium. These cells were washed once with PBS and resuspended in RPMI medium without WHEHI-3 conditioned medium and seeded into each well of 96-well microtiter plates at a density of 5 X10⁴ cells per well in the presence of the present compound or TPO. After incubation at 37°C for 20 hours in the 5% CO₂ incubator, WST-1 reagent (Takara Biomedicals, Japan) was added to each wells and the cells were further incubated for 4 hours. The absorbance at 450 nm was measured. Table 7 exemplifies the ED₅₀ for tested compounds of the present invention, wherein the ED₅₀ is the half concentration of the concentration showing the maximum thrombopoietic activity.

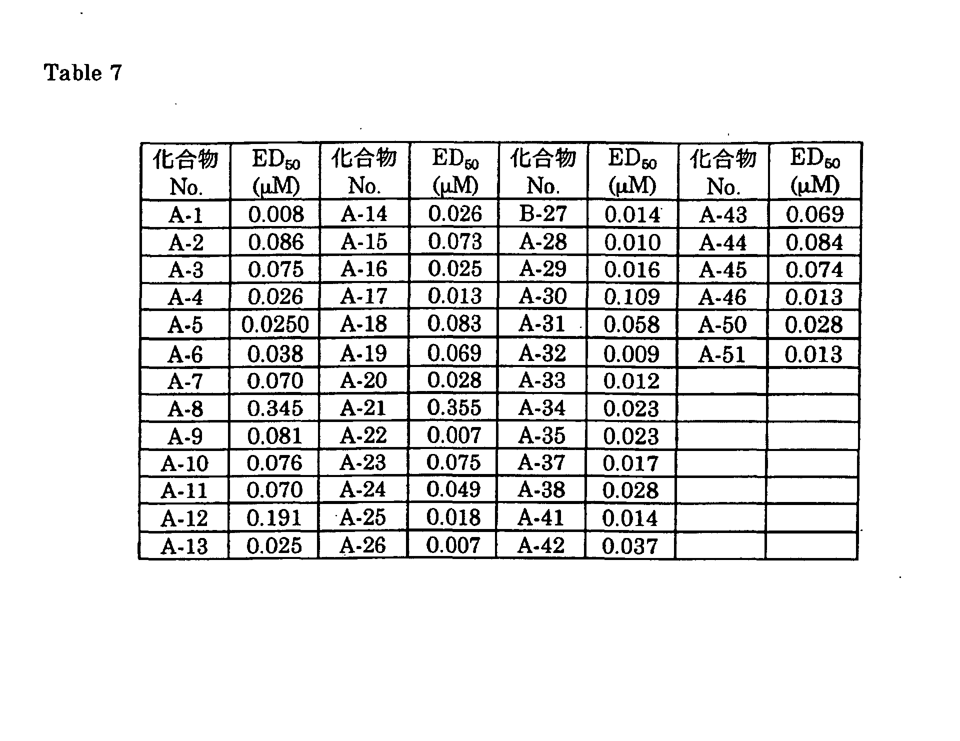

### Formulation example

### Formulation example 1

Granules are prepared using the following ingredients.

| | | |
|---|---|---|
| Ingredients | The compound represented by the formula (I) | 10 mg |
| | Lactose | 700 mg |
| | Corn starch | 274 mg |
| | HPC-L | 16 mg |
| | | 1000 mg |

The compound represented by the formula (I) and lactose are made pass through a 60 mesh sieve. Corn starch is made pass through a 120 mesh sieve. They are mixed by a twin shell blender. An aqueous solution of HPC-L (low mucosity hydroxypropylcellulose) is added to the mixture and the resulting mixture is kneaded, granulated (by the extrusion with pore size 0.5 to 1 mm mesh), and dried. The dried granules thus obtained are sieved by a swing sieve (12/60 mesh) to yield the granules.

### Formulation 2

Powders for filling capsules are prepared using the following ingredients.

| | | |
|---|---|---|
| Ingredients | The compound represented by the formula (I) | 10 mg |
| | Lactose | 79 mg |
| | Corn starch | 10 mg |
| | Magnesium stearate | 1 mg |
| | | 100 mg |

The compound represented by the formula (I) and lactose are made pass through a 60 mesh sieve. Corn starch is made pass through a 120 mesh sieve. These ingredients and magnesium stearate are mixed by a twin shell blender. 100 mg of the 10-fold trituration is filled into a No. 5 hard gelatin capsule.

### Formulation 3

Granules for filling capsules are prepared using the following ingredients.

| | | |
|---|---|---|
| Ingredients | The compound represented by the formula (I) | 15 mg |
| | Lactose | 90 mg |
| | Corn starch | 42 mg |
| | HPC-L | 3 mg |
| | | 150 mg |

The compound represented by the formula (I) and lactose are made pass through a 60 mesh sieve. Corn starch is made pass through a 120 mesh sieve. After mixing them, an aqueous solution of HP C-L is added to the mixture and the resulting mixture is kneaded, granulated, and dried. After the dried granules are lubricated, 150 mg of that are filled into a No. 4 hard gelatin capsule.

### Formulation 4

Tablets are prepared using the following ingredients.

| | | |
|---|---|---|
| Ingredients | The compound represented by the formula (I) | 10 mg |
| | Lactose | 90 mg |
| | Microcrystal cellulose | 30 mg |
| | CMC-Na | 15 mg |
| | Magnesium stearate | 5 mg |
| | | 150 mg |

The compound represented by the formula (I), lactose, microcrystal cellulose, and CMC-Na (carboxymethylcellulose sodium salt) are made pass through a 60 mesh sieve and then mixed. The resulting mixture is mixed with magnesium stearate to obtain the mixed powder for the tablet formulation. The mixed powder is compressed to yield tablets of 150 mg.

### Industrial Applicability

The compounds of the present invention have thrombopoietin receptor agonism and are useful as the treating or preventing agent for hemopathy accompanied with unusual count of platelet, for example, thrombocytopenia and the like.

## Claims

1. A pharmaceutical composition exhibiting thrombopoietin receptor agonism which contains as an active ingredient a compound of the general formula (I): 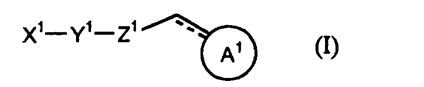 wherein X¹ is a group represented by the formula: 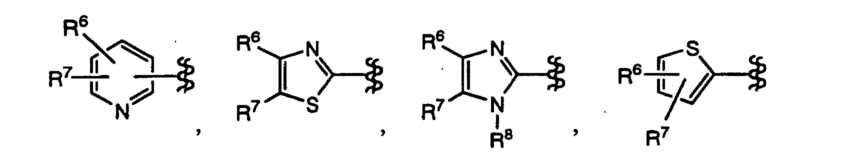 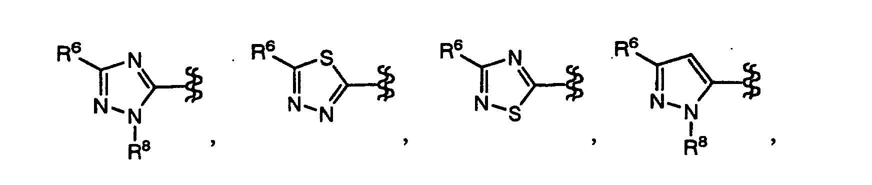 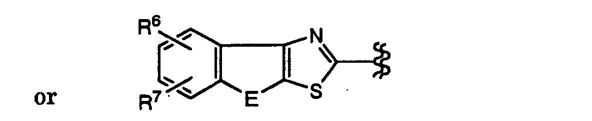 wherein E is -CH₂-, -(CH₂)₂-, -(CH₂)₃-, -O-CH₂-, or -S-CH₂-; one of R⁶ and R⁷ is a group represented by the formula: 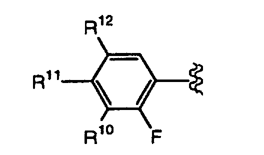 wherein R¹⁰, R¹¹, and R¹² are each independently hydrogen atom, alkyl optionally substituted with one or more substituent(s) selected from substituent group A, cycloalkyl, alkyloxy optionally substituted with one or more substituent(s) selected from substituent group A, alkylthio, halogen atom, phenyl optionally substituted with one or more substituent(s) selected from substituent group B, heteroaryl optionally substituted with one or more substituent(s) selected from substituent group B, or non-aromatic heterocyclic group optionally substituted with one or more substituent(s) selected from substituent group B, substituent group A consists of cycloalkyl, hydroxy, optionally substituted alkyloxy, halogen atom, carboxy, lower alkyloxycarbonyl, aryloxycarbonyl, optionally substituted amino, optionally substituted aminocarbonyl, phenyl optionally substituted with one or more substituent(s) selected from substituent group B, non-aromatic heterocyclic group, and heteroaryl,
substituent group B consists of hydroxy, alkyl, halogen atom, halo(lower)alkyl, carboxy, lower alkyloxycarbonyl, alkyloxy, optionally substituted amino, non-aromatic heterocyclic group, and heteroaryl;
the other of R⁶ and R⁷ is hydrogen atom, optionally substituted lower alkyl, carboxy, lower alkyloxycarbonyl, halogen atom, optionally substituted aminocarbonyl, optionally substituted heteroaryl, or optionally substituted aryl; R⁸ is hydrogen atom or lower alkyl;
Y¹ is NR^{A}CO-(CR^{C}R^{D})₀₋₂-, -NR^{A}CO-(CH₂)₀₋₂-V-, -NR^{A}CO-CR^{C}=CR^{D}-, -V- (CH₂)₁-₅-NR^{A}CO-(CH₂)₀₋₂-, -V-(CH₂)₁₋₅- CONR^{A}-(CH₂)₀₋₂-, -CONR^{A}-(CH₂)₀₋₂-, -(CH₂)₀₋₂-NR^{A}-SO₂-(CH₂)_{0.2}-, -(CH₂)_{0.2}-SO₂-NR^{A}-(CH₂)_{0.2}-, -NR^{A}-(CH₂)_{0.2}-, -NR^{A}-CO-NR^{A}-, -NR^{A}-CS-NR^{A}-,-N=C(-SR^{A})-NR^{A}-, -NR^{A}CSNR^{A}CO-, -N=C(-SR^{A})-NR^{A}CO-, -NR^{A}-(CH₂)₁₋₂-NR^{A}-CO-,-NR^{A}CONR^{A}NR^{B}CO-, or -N=C(-NR^{A}R^{A})-NR^{A}-CO- wherein R^{A} is each independently hydrogen atom or lower alkyl; R^{B} is hydrogen atom or phenyl; R^{C} and R^{D} are each independently hydrogen atom, halogen atom, optionally substituted lower alkyl, optionally substituted lower alkyloxy, optionally substituted lower alkylthio, optionally substituted lower alkenyl, optionally substituted lower alkynyl, optionally substituted aryl, optionally substituted heteroaryl, optionally substituted cycloalkyl, optionally substituted aralkyl, optionally substituted heteroarylalkyl, optionally substituted non-aromatic heterocyclic group, or optionally substituted amino; V is oxygen atom or sulfur atom;
Z¹ is optionally substituted arylene, optionally substituted heteroarylene, optionally substituted non-aromatic heterocyclicdiyl, or optionally substituted cycloalkylene;
A¹ ring is a ring represented by the formula: 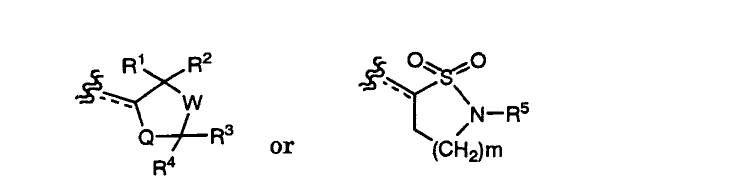 wherein R¹ and R² are hydrogen atom or taken together to be oxygen atom or sulfur atom; R³ and R⁴ are hydrogen atom or taken together to be oxygen atom or sulfur atom; R⁵ is hydrogen atom or lower alkyl; Q and W are each independently -O-, -S-, -N(R^{F})-wherein R^{F} is hydrogen atom or lower alkyl, or -CH₂-; m is 1,2, or 3; a broken line (---) represents the presence or absence of a bond;
a broken line (---) represents the presence or absence of a bond;
provided that R¹⁰, R¹¹, and R¹² are not hydrogen atom at the same time; when R¹⁰ and R¹¹ are hydrogen, R¹² is not fluoro; when R¹⁰ is hydrogen, R¹¹ and R¹² are not fluoro;
its prodrug, or their pharmaceutically acceptable salt, or solvate thereof.

2. A pharmaceutical composition exhibiting thrombopoietin receptor agonism of claim 1, wherein Y¹ is -NHCO-, -CONH-, -NHCH₂-, -NHCO-CH=CH-, or -NHSO₂-.

3. A pharmaceutical composition exhibiting thrombopoietin receptor agonism of claim 1 or 2, wherein Z¹ is halogen atom or 1,4-phenylene optionally substituted with lower alkyl.

4. A pharmaceutical composition exhibiting thrombopoietin receptor agonism of any one of claims 1 to 3, wherein A¹ ring is a group represented by the formula: 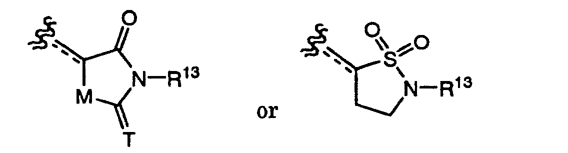 wherein R¹³ is hydrogen atom or lower alkyl; M is -S-, -O-, -N(R^{E})- wherein R^{E} is hydrogen atom or lower alkyl; or -CH₂-; T is oxygen atom or sulfur atom; a broken line (---) represents the presence or absence of a bond.

5. A pharmaceutical composition exhibiting thrombopoietin receptor agonism of any one of claims 1 to 4, wherein a broken line (---) represents the presence of a bond.

6. A pharmaceutical composition exhibiting thrombopoietin receptor agonism of any one of claims 1 to 5, which is a platelet production modifier.

7. Use of a compound of any one of claims 1 to 5, for preparation of a medicine for modifying platelet production.

8. A method for modifying platelet production of a mammal, including a human, which comprises administration to said mammal of a compound of any one of claims 1 to 5 in a therapeutically effective amount.

9. A compound represented by the general formula (II): 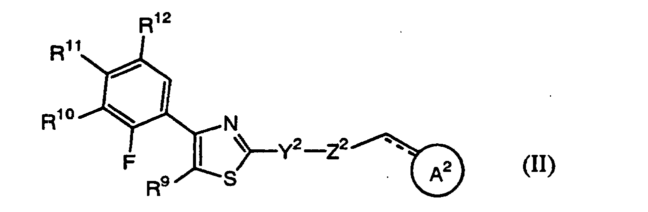 wherein R⁹ is hydrogen atom, optionally substituted lower alkyl; carboxy, lower alkyloxycarbonyl, halogen atom, or optionally substituted aminocarbonyl;
R¹⁰, R¹¹, and R¹² are each independently hydrogen atom, alkyl optionally substituted with one or more substituent(s) selected from substituent group A, cycloalkyl, alkyloxy optionally substituted with one or more substituent(s) selected from substituent group A, alkylthio, halogen atom, phenyl optionally substituted with one or more substituent(s) selected from substituent group B, heteroaryl optionally substituted with one or more substituent(s) selected from substituent group B, or non-aromatic heterocyclic group optionally substituted with one or more substituent(s) selected from substituent group B,
substituent group A consists of cycloalkyl, hydroxy, optionally substituted alkyloxy, halogen atom, carboxy, lower alkyloxycarbonyl, aryloxycarbonyl, optionally substituted amino, optionally substituted aminocarbonyl, phenyl optionally substituted.with one or more substituent(s) selected from substituent group B, non-aromatic heterocyclic group, and heteroaryl,
substituent group B consists of hydroxy, alkyl, halogen atom, halo(lower)alkyl, carboxy, lower alkyloxycarbonyl, alkyloxy, optionally substituted amino, non-aromatic heterocyclic group, and heteroaryl;
Y² is -NR^{A}CO-(CR^{C}R^{D})₀₋₂-, -NR^{A}-CO-(CH₂)₀₋₂-V-, -NR^{A}CO-CR^{C}=CR^{D}-, -V-(CH₂)₁₋₅-NR^{A}CO-(CH₂)₀₋₂-, -V-(CH₂)₁₋₅-CONR^{A}-(CH₂)₀₋₂-, -CONR^{A}-(CH₂)₀₋₂-,-(CH₂)₀₋₂-NR^{A}-SO₂-(CH₂)₀₋₂-, -(CH₂)₀₋₂-SO₂-NR^{A} -(CH₂)₀₋₂-, -NR^{A}-(CH₂)₀₋₂-, -NR^{A}-CO-NR^{A}-, -NR^{A}-CS-NR^{A}-,-N=C(-SR^{A})-NR^{A}-, -NR^{A}CSNR^{A}CO-, -N=C(-SR^{A})-NR^{A}CO-, -NR^{A}-(CH₂)₁₋₂-NR^{A}-CO-,-NR^{A}CONR^{A}NR^{B}CO-, or -N=C(-NR^{A}R^{A})-NR^{A}-CO- wherein R^{A} is each independently hydrogen atom or lower alkyl; R^{B} is hydrogen atom or phenyl; R^{C} and R^{D} are each independently hydrogen atom, halogen atom, optionally substituted lower alkyl, optionally substituted lower alkyloxy, optionally substituted lower alkylthio, optionally substituted lower alkenyl, optionally substituted lower alkynyl, optionally substituted aryl, optionally substituted heteroaryl, optionally substituted cycloalkyl, optionally substituted aralkyl, optionally substituted heteroarylalkyl, optionally substituted non-aromatic heterocyclic group, or optionally substituted amino; V is oxygen atom or sulfur atom;
Z² is optionally substituted phenylene, optionally substituted 2,5-pyridinediyl, optionally substituted 2,5-thiophenediyl, or optionally substituted 2,5-furandiyl;
A² ring is a ring represented by the formula: 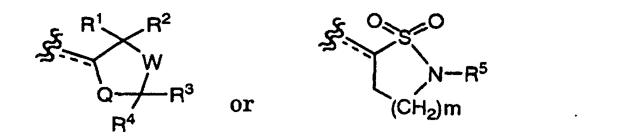 wherein R¹ and R² are hydrogen atom or taken together to be oxygen atom or sulfur atom; R³ and R⁴ are hydrogen atom or taken together to be oxygen atom or sulfur atom; R⁵ is hydrogen atom or lower alkyl; Q and W are each independently -O-, -S-, -N(R^{F})-wherein R^{F} is hydrogen atom or lower alkyl, or -CH₂-; m is 1,2, or 3; a broken line (---) represents the presence or absence of a bond;
a broken line (---) represents the presence or absence of a bond;
provided that R¹⁰, R¹¹, and R¹² are not hydrogen atom at the same time; when R¹⁰ and R¹¹ are hydrogen, R¹² is not fluoro; when R¹⁰ is hydrogen, R¹¹ and R¹² are not fluoro;
its prodrug, or their pharmaceutically acceptable salt, or solvate thereof.

10. A compound of claim 9, wherein Y² is -NHCO-, -CONH-, -NHCH₂-, -NHCO-CH=CH-, or -NHSO₂-;
its prodrug, or their pharmaceutically acceptable salt, or solvate thereof.

11. A compound of claim 9, wherein Y² is -NHCO-;
its prodrug, or their pharmaceutically acceptable salt, or solvate thereof.

12. A compound of any one of claims 9 to 11, wherein Z² is halogen atom or 1,4-phenylene optionally substituted with lower alkyl;
its prodrug, or their pharmaceutically acceptable salt, or solvate thereof.

13. A compound of any one of claims 9 to 12, wherein A² ring is a group represented by the formula: 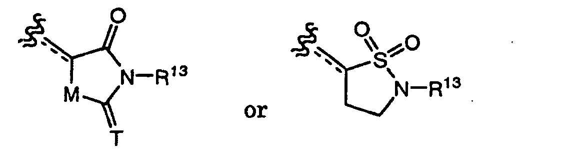 wherein R¹³ is hydrogen atom or lower alkyl; M is -S-, -O-, -N(R^{E})- wherein R^{E} is hydrogen atom or lower alkyl; or -CH₂-; T is oxygen atom or sulfur atom; a broken line (---) represents the presence or absence of a bond;
its prodrug, or their pharmaceutically acceptable salt, or solvate thereof.

14. A compound of any one of claims 9 to 13, wherein a broken line (---) represents the presence of a bond;
its prodrug, or their pharmaceutically acceptable salt, or solvate thereof.

15. A compound of the general formula (III): 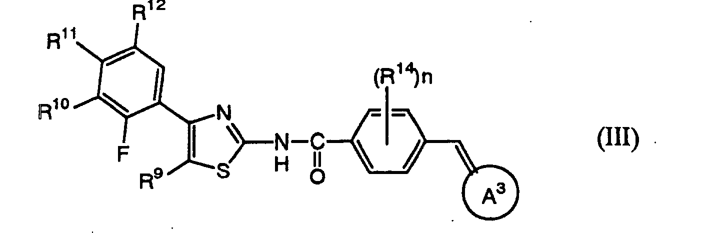 wherein R⁹ is hydrogen atom, optionally substituted lower alkyl; carboxy, lower alkyloxycarbonyl, halogen atom, or optionally substituted aminocarbonyl;
R¹⁰ is alkyl optionally substituted with one or more substituent(s) selected from substituent group A, cycloalkyl, alkyloxy optionally substituted with one or more substituent(s) selected from substituent group A, alkylthio, halogen atom, phenyl optionally substituted with one or more substituent(s) selected from substituent group B, heteroaryl optionally substituted with one or more substituent(s) selected from substituent group B, or non-aromatic heterocyclic group optionally substituted with one or more substituent(s) selected from substituent group B,
R¹¹ and R¹² are each independently hydrogen atom, alkyl optionally substituted with one or more substituent(s) selected from substituent group A, cycloalkyl, alkyloxy optionally substituted with one or more substituent(s) selected from substituent group A, alkylthio, halogen atom, phenyl optionally substituted with one or more substituent(s) selected from substituent group B, heteroaryl optionally substituted with one or more substituent(s) selected from substituent group B, or non-aromatic heterocyclic group optionally substituted with one or more substituent(s) selected from substituent group B,
substituent group A consists of cycloalkyl, hydroxy, optionally substituted alkyloxy, halogen atom, carboxy, lower alkyloxycarbonyl, aryloxycarbonyl, optionally substituted amino, optionally substituted aminocarbonyl, phenyl optionally substituted with one or more substituent(s) selected from substituent group B, non-aromatic heterocyclic group, and heteroaryl, substituent group B consists of hydroxy, alkyl, halogen atom, halo(lower)alkyl, carl lower alkyloxycarbonyl, alkyloxy, optionally substituted amino, non-aron heterocyclic group, and heteroaryl;
R¹⁴ is each independently lower alkyl, halogen atom, halo(lower)alkyl, lower alkyloxy, halo(lower)alkyloxy, or hydroxy;
n is an integer of 0 to 2;
A² ring is a group represented by the formula: 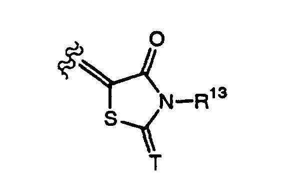 wherein R¹³ is hydrogen atom or lower alkyl; T is oxygen atom or sulfur atom; its prodrug, or their pharmaceutically acceptable salt, or solvate thereof.

16. A compound of claim 15, wherein R¹⁰ is alkyl optionally substituted with one or more substituent(s) selected from substituent group A, alkyloxy, halo(lower)alkyloxy, or phenyl optionally substituted with one or more substituent(s) selected from substituent group B; R¹¹ is hydrogen atom, halo(lower)alkyl, or halo(lower)alkyloxy; R¹² is hydrogen atom or fluoro;
substituent group A consists of cycloalkyl, hydroxy, optionally substituted alkyloxy, halogen atom, carboxy, lower alkyloxycarbonyl, aryloxycarbonyl, optionally substituted amino, optionally substituted aminocarbonyl, phenyl optionally substituted with one or more substituent(s) selected from substituent group B, non-aromatic heterocyclic group, and heteroaryl,
substituent group B consists of hydroxy, alkyl, halogen atom, halo(lower)alkyl, carboxy, lower alkyloxycarbonyl, alkyloxy, optionally substituted amino, non-aromatic heterocyclic group, and heteroaryl;
its prodrug, or their pharmaceutically acceptable salt, or solvate thereof.

17. A pharmaceutical composition containing as an active ingredient a compound of any one of claims 9 to 16.

18. A pharmaceutical composition exhibiting thrombopoietin receptor agonism, which contains as an active ingredient a compound of any one of claims 9 to 16.

19. A platelet production modifier containing as an active ingredient a compound of any one of claims 9 to 16.

20. Use of a compound of any one of claims 9 to 16, for preparation of a medicine for modifying platelet production.
